# EUROPEAN PATENT APPLICATION

(11) **EP 3 960 239 A1**
(43) Date of publication of application: **02.03.2022**
(21) Application number: 20193139.1
(22) Date of filing: 27.08.2020
(51) Int. Cl.: A61P 31/00, A61K 38/55, A61P 31/12, C07C 233/15, C07C 233/19, C07C 233/38, C07C 233/88, C07C 323/29, C07D 211/28, C07D 311/74

(54) **PROTEASES INHIBITORS FOR USE IN THE TREATMENT OF INFECTIONS CAUSED BY SARS CORONAVIRUSES**

(71) Applicant: Universität Konstanz, 78464 Konstanz (DE)
(72) Inventor: Böttcher, Thomas, 78467 Konstanz (DE); Peñalver, Lilian, 78464 Konstanz (DE)
(74) Representative: Müller-Boré & Partner Patentanwälte PartG mbB

(57) **Abstract**

The present invention relates to a compound which can be used in the treatment of infections caused by SARS coronaviruses, e.g. by blocking the active site of the proteases 3CL^{pro} and PL^{pro}. The compound can be used as inhibitor or label as such or can be used in screening methods for profiling other inhibitors. Moreover, the present invention relates to a method of producing the compound.

## Description

The present invention relates to a compound which can be used in the treatment of infections caused by SARS coronaviruses, e.g. by blocking the active site of the proteases 3CL^{pro} and PL^{pro}. The compound can be used as inhibitor or label as such or can be used in screening methods for profiling other inhibitors. Moreover, the present invention relates to a method of producing the compound.

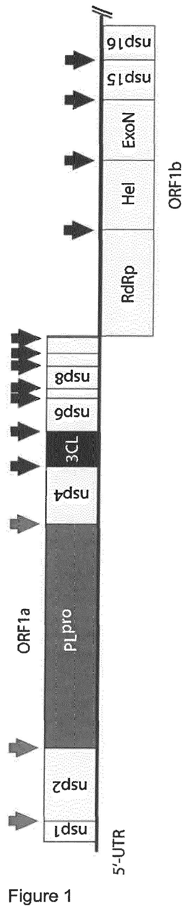

SARS-CoV-2 is an important virus that is responsible for the COVID-19 pandemic. The genome of the ssRNA-virus contains at least 10 open reading frames (ORFs) of which ORF1a/b encodes the proteins required for the replication in the eukaryotic host cell and is translated via a ribosomal -1 frame shifting mechanism into a polyprotein (Fig. 1). The polyprotein is processed by the two virus-encoded proteases PL^{pro} and 3CL^{pro}, a papain-like and a 3C-like protease, respectively. The activity of these proteases is, thus, essential for the reproduction of SARS-CoV-2 in its host cells. PL^{pro} additionally protects the virus from the innate immune response by its deubiquitinating activity.

It is very burdensome and time consuming to find suitable enzyme inhibitors, particularly inhibitors having cell permeability. However, time is a critical factor during ongoing and fast spreading diseases, such as COVID-19. Accordingly, there is a need for techniques which are able to screen fast and reliably for potent inhibitors, which have cell permeability and low probability of off-targeting.

Thus, the technical problem underlying the present invention is to provide a method which is able to screen fast and reliably for suitable inhibitor structures, which have cell permeability and low probability of off-targeting, as well as resulting structures, which can be further developed to potent inhibitors.

The solution to the above technical problem is achieved by the embodiments characterized in the claims.

In particular, the present invention relates to a compound represented by the following general formula (1), 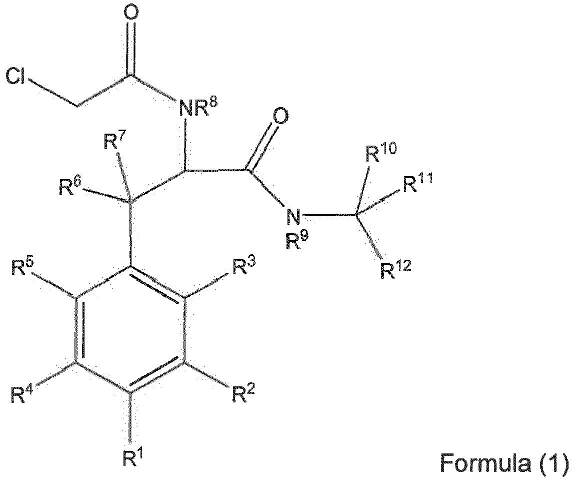
wherein one of R¹ to R⁵ is selected from the group consisting of an azide group, an alkyl group, a cycloalkyl group, an alkenyl group, a cycloalkenyl group, an alkynyl group, a biotin group, an aryl group, and a heteroaryl group, -NX¹X², -OX³, -SX⁴,-C(O)X⁵, -C(O)NX⁶X⁷, -COOX⁸, and -SO₃X⁹, wherein X¹ to X⁹ are independently selected from the group consisting of a hydrogen atom, an alkyl group, a cycloalkyl group, an alkenyl group, a cycloalkenyl group, an alkynyl group, an aryl group, and a heteroaryl group, wherein the one of R¹ to R⁵ contains an azide group, an alkynyl group, a biotin group, or a fluorophoric group;
the remaining four of R¹ to R⁵ are independently selected from the group consisting of a hydrogen atom, an alkyl group, a cycloalkyl group, an alkenyl group, a cycloalkenyl group, an alkynyl group, an aryl group, a heteroaryl group, a halogen atom, -NE¹E², -NO₂, -CN, -OE³, -SE⁴, -C(O)E⁵, -C(O)NE⁶E⁷, -COOE⁸, and -SO₃E⁹, wherein E¹ to E⁹ are independently selected from the group consisting of a hydrogen atom, an alkyl group, a cycloalkyl group, an alkenyl group, a cycloalkenyl group, an alkynyl group, an aryl group, and a heteroaryl group, and wherein R¹ to R⁵ may bind to each other to form one or more rings;
R⁶, R⁷, and R¹⁰ to R¹² are independently selected from the group consisting of a hydrogen atom, an alkyl group, a cycloalkyl group, an alkenyl group, a cycloalkenyl group, an alkynyl group, an aryl group, a heteroaryl group, a halogen atom, -NA¹A²,-NO₂, -CN, -OA³, -SA⁴, -C(O)A⁵, -C(O)NA⁶A⁷, -COOA⁸, and -SO₃A⁹, wherein A¹ to A⁹ are independently selected from the group consisting of a hydrogen atom, an alkyl group, a cycloalkyl group, an alkenyl group, a cycloalkenyl group, an alkynyl group, an aryl group, and a heteroaryl group, and wherein R⁶ and R⁷ and/or R¹⁰ to R¹² may bind to each other to form one or more rings; and
R⁸ and R⁹ are independently selected from the group consisting of a hydrogen atom, an alkyl group, a cycloalkyl group, an alkenyl group, a cycloalkenyl group, an alkynyl group, an aryl group, and a heteroaryl group;
or a pharmaceutically acceptable salt thereof; and
wherein the alkyl groups, the cycloalkyl groups, the alkenyl groups, the cycloalkenyl groups, the alkynyl groups, the aryl groups, and the heteroaryl groups may independently be (further) substituted or unsubstituted and the alkyl groups, the alkenyl groups, and the alkynyl groups may independently be branched or linear.

The compound according to the present invention can be used as an inhibitor or marker of proteases PL^{pro} and 3CL^{pro} of a SARS coronavirus, particularly SARS-CoV-2, and can label these polypeptides, preferably in the active sites thereof. Furthermore, the compound according to the present invention can be used in screening for other potent inhibitors of said proteases. The compound according to the present invention and/or the newly found potent inhibitors preferably have cell permeability and low probability of off-targeting. The compound according to the present invention is preferably able to target the nucleophilic active site cysteine of each protease. It is preferably possible to profile the activity state of the proteases in live cells and to use the compound as a chemical tool to screen for new inhibitors. The compound preferably can be used to screen for inhibitors of the protease 3CL^{pro} prior to activation by autocleavage of its N-terminus. Moreover, the compound according to the present invention can be used directly as inhibitor and scaffold for structural refinements. Preferably, the compound according to the present invention has specificity for labeling of only the active site residue and selectivity for labeling only the target protein in the background of an entire proteome.

Herein, the term "fluorophoric group" means a group, which provides fluorescent properties to the respective compound. Examples of fluorophoric groups are rhodamine, tetramethylrhodamine (TAMRA), 4,4-difluoro-4-bora-3a,4a-diaza-s-indacenes (BODIPY-dyes), fluorescein, cyanine dyes (e.g. Cy3, Cy5, Cy7), sulforhodamines (Texas red), Alexa Fluor dyes (e.g. AF546, AF555, AF594, AF647), and coumarin dyes. Preferably, the fluorophoric group is rhodamine or tetramethylrhodamine (TAMRA), more preferably tetramethylrhodamine (TAMRA).

If not stated otherwise, the following definitions apply to the terms "halogen", "alkyl group", "cycloalkyl group", "alkenyl group", "cycloalkenyl group", "alkynyl group", "aryl group", and "heteroaryl group". Herein the term "halogen" refers particularly to fluorine atoms, chlorine atoms, bromine atoms, and iodine atoms, preferably fluorine atoms and chlorine atoms, most preferably fluorine atoms. The term "alkyl group" refers particularly to a branched or linear alkyl group having 1 to 20, preferably 1 to 12, more preferably 1 to 6, and most preferably 1 to 4 carbon atoms, which can be substituted or unsubstituted. Examples of alkyl groups represent methyl groups, ethyl groups, propyl groups, isopropyl groups, butyl groups, isobutyl groups, tert-butyl groups, pentyl groups, hexyl groups, and heptyl groups. The term "cycloalkyl group" refers particularly to a cycloalkyl group having 3 to 10, preferably 4 to 8, more preferably 5 or 6, and most preferably 6 carbon atoms, which can be substituted or unsubstituted. Examples of cycloalkyl groups represent cyclobutyl groups, cyclopentyl groups, and cyclohexyl groups. The term "alkenyl group" refers particularly to a branched or linear alkenyl group having 2 to 20, preferably 2 to 12, more preferably 2 to 6, and most preferably 2 to 4 carbon atoms, which can be substituted or unsubstituted. Examples of alkenyl groups represent vinyl groups and allyl groups. The term "cycloalkenyl group" refers particularly to a cycloalkenyl group having 4 to 10, preferably 5 to 8, more preferably 5 or 6, and most preferably 6 carbon atoms, which can be substituted or unsubstituted. Examples of cycloalkenyl groups represent cyclopentenyl groups, cyclopentadienyl groups, cyclohexyl groups, and cyclohexadienyl groups. The term "alkynyl group" refers particularly to a branched or linear alkynyl group having 2 to 20, preferably 2 to 12, more preferably 2 to 6, and most preferably 2 to 4 carbon atoms, which can be substituted or unsubstituted. Examples of alkynyl groups represent ethynyl groups, 1-propynyl groups, and propargyl groups. The term "aryl group" refers particularly to an aryl group consisting of 1 to 6, preferably 1 to 4, more preferably 1 to 3 aromatic rings, and most preferably 1 ring, which can be substituted or unsubstituted.

Examples of aryl groups represent phenyl groups, anthracenyl or naphthyl groups. The term "heteroaryl group" refers particularly to a heteroaryl group consisting of 1 to 6, preferably 1 to 4, more preferably 1 to 3 aromatic rings including heteroatoms, which can be substituted or unsubstituted. Heteroatoms, which are present in heteroaryl groups are for example N, O and S. Examples of heteroaryl groups represent pyridyl groups, pyrimidinyl groups, thienyl groups, furyl groups, or pyrrolyl groups.

According to the present invention, the alkyl groups, the cycloalkyl groups, the alkenyl groups, the cycloalkenyl groups, the alkynyl groups, the aryl groups, and the heteroaryl groups may be substituted or unsubstituted. The potential substituents are not specifically limited. Accordingly, instead of hydrogen atoms any substituent known in the prior art can be bonded to the further positions of the corresponding groups. For example, the potential substituents may be selected from the group consisting of a branched or linear alkyl group having 1 to 6 carbon atoms, a cycloalkyl group having 4 to 8 carbon atoms, a branched or linear alkenyl group having 2 to 6 carbon atoms, a cycloalkenyl group having 4 to 8 carbon atoms, a branched or linear alkynyl group having 2 to 6 carbon atoms, an aryl group having 1 to 3 aromatic rings, a heteroaryl group having 1 to 3 aromatic rings including heteroatoms, a halogen atom, -NL¹L², -NO₂, -CN, -OL³, -C(O)L⁴, -C(O)NL⁵L⁶, -COOL⁷, and -SO₃L⁸, wherein L¹ to L⁸ are each independently selected from a hydrogen atom, a branched or linear alkyl group having 1 to 6 carbon atoms, a cycloalkyl group having 4 to 8 carbon atoms, a branched or linear alkenyl group having 2 to 6 carbon atoms, a cycloalkenyl group having 4 to 8 carbon atoms, a branched or linear alkynyl group having 2 to 6 carbon atoms, an aryl group having 1 to 3 aromatic rings, a heteroaryl group having 1 to 3 aromatic rings including heteroatoms. Accordingly, examples of substituted alkyl groups are aralkyl groups or alkyl groups substituted with e.g. halogen atoms, such as e.g. a trifluoromethyl group, or any other of the above-mentioned substituents. The term "aralkyl group" refers particularly to an alkyl group wherein one or more hydrogen atoms, preferably terminal hydrogen atoms of the alkyl chain, are replaced by aryl or heteroaryl groups. Examples of aralkyl groups represent benzyl groups or 1- or 2-phenylethyl groups. Preferably, the potential substituents are selected from the group consisting of a branched or linear alkyl group having 1 to 6 carbon atoms, a branched or linear alkenyl group having 2 to 6 carbon atoms, a branched or linear alkynyl group having 2 to 6 carbon atoms, a halogen atom, -NH₂, -NHCH₃, -N(CH₃)₂, -NO₂, -OH,-OCH₃, -OEt, -C(O)H, -C(O)CH₃, -C(O)Et, and -COOH, more preferably selected from the group consisting of a branched or linear alkyl group having 1 to 6 carbon atoms, a halogen atom, -NH₂, -NHCH₃, -N(CH₃)₂, -NO₂, -OH, -OCH₃, and -OEt. Moreover, one or more tetravalent carbon atoms (together with the hydrogen atoms bonded thereto), when present, in each of the alkyl groups, the cycloalkyl groups, the alkenyl groups, the cycloalkenyl groups, and the alkynyl groups may each independently be substituted by a member selected from the group consisting of O, (OCH₂CH₂)ₙO, S, (SCH₂CH₂)ₘS, C(O), C(O)O, NL⁹, and C(O)NL¹⁰, preferably O, (OCH₂CH₂)ₙO, C(O)O, and C(O)NL¹⁰, wherein n and m are each independently an integer from 1 to 6. Accordingly, for example an alkyl group may be interrupted by e.g. one or more PEG linkers and/or amide bonds, and an alkenyl group may contain a C(O) group, such as in an acryloyl group. The way the groups are introduced instead of a carbon atom is not specifically limited. For example, a carbon atom may be substituted by C(O)O in the sense of -C(O)O- or -OC(O)- and by C(O)NL¹⁰ in the sense of -C(O)NL¹⁰- or-NL¹⁰C(O)-. According to the present invention, L⁹ and L¹⁰ are each independently selected from the group consisting of a hydrogen atom, a branched or linear alkyl group having 1 to 6 carbon atoms, a cycloalkyl group having 4 to 8 carbon atoms, a branched or linear alkenyl group having 2 to 6 carbon atoms, a cycloalkenyl group having 4 to 8 carbon atoms, a branched or linear alkynyl group having 2 to 6 carbon atoms, an aryl group having 1 to 3 aromatic rings, a heteroaryl group having 1 to 3 aromatic rings including heteroatoms, -OG¹, -C(O)G², -C(O)NG³G⁴, -COOG⁵, and -SO₂G⁶. In a preferred embodiment, L⁹ and L¹⁰ are each independently selected from the group consisting of a hydrogen atom, a branched or linear alkyl group having 1 to 6 carbon atoms, an aryl group having 1 to 3 aromatic rings, -C(O)G², and -SO₂G⁶. Most preferably, L⁹ and L¹⁰ are each independently selected from the group consisting of a hydrogen atom and a branched or linear alkyl group having 1 to 6 carbon atoms. According to the present invention, G¹ to G⁶ are each independently selected from the group consisting of a hydrogen atom, a branched or linear alkyl group having 1 to 6 carbon atoms, a cycloalkyl group having 4 to 8 carbon atoms, a branched or linear alkenyl group having 2 to 6 carbon atoms, a cycloalkenyl group having 4 to 8 carbon atoms, a branched or linear alkynyl group having 2 to 6 carbon atoms, an aryl group having 1 to 3 aromatic rings, a heteroaryl group having 1 to 3 aromatic rings including heteroatoms. In a preferred embodiment, G¹ to G⁶ are each independently selected from the group consisting of a hydrogen atom, a branched or linear alkyl group having 1 to 6 carbon atoms, an aryl group having 1 to 3 aromatic rings.

If not stated otherwise, the alkyl groups, the cycloalkyl groups, the alkenyl groups, the cycloalkenyl groups, the alkynyl groups, the aryl groups, and the heteroaryl groups are preferably unsubstituted. Moreover, if not stated otherwise, the alkyl groups, the alkenyl groups, and the alkynyl groups are preferably linear.

According to the present invention, one of R¹ to R⁵ is selected from the group consisting of an azide group, an alkyl group, a cycloalkyl group, an alkenyl group, a cycloalkenyl group, an alkynyl group, a biotin group, an aryl group, and a heteroaryl group, -NX¹X², -OX³, -SX⁴, -C(O)X⁵, -C(O)NX⁶X⁷, -COOX⁸, and -SO₃X⁹, wherein X¹ to X⁹ are independently selected from the group consisting of a hydrogen atom, an alkyl group, a cycloalkyl group, an alkenyl group, a cycloalkenyl group, an alkynyl group, an aryl group, and a heteroaryl group, wherein the one of R¹ to R⁵ contains an azide group, an alkynyl group, a biotin group, or a fluorophoric group. By means of said one group of R¹ to R⁵, it is possible to identify the compound according to the present invention by analytic methods, such as spectrophotometry. Said one group of R¹ to R⁵ contains a fluorophoric group or a biotin group, each of which can directly be used for analytic purposes, or contains an azide group or an alkynyl group, which can be converted to a fluorophoric group or a biotin group, by reacting said groups with an alkyne or an azide, respectively, via a click-chemical reaction.

Preferably, the one of R¹ to R⁵ is selected from the group consisting of an azide group, an alkyl group, an alkenyl group, an alkynyl group, an aryl group, a heteroaryl group, - NX¹X², -OX³, and -SX⁴, more preferably from the group consisting of an azide group, an alkynyl group, and -OX³, more preferably from an alkynyl group and -OX³. More preferably, the one of R¹ to R⁵ is -OX³.

Preferably, X¹ to X⁹ are independently selected from the group consisting of a hydrogen atom, an alkyl group, an alkenyl group, an alkynyl group, an aryl group, and a heteroaryl group, more preferably from the group consisting of an alkyl group, an alkynyl group, and an aryl group. More preferably, X¹ to X⁹ are an alkynyl group, more preferably a propargyl group. More preferably, the one of R¹ to R⁵ is -OX³, wherein X³ is an alkynyl group. Most preferably, the one of R¹ to R⁵ is -OX³, wherein X³ is a propargyl group.

According to the present invention, the remaining four of R¹ to R⁵ are independently selected from the group consisting of a hydrogen atom, an alkyl group, a cycloalkyl group, an alkenyl group, a cycloalkenyl group, an alkynyl group, an aryl group, a heteroaryl group, a halogen atom, -NE¹E², -NO₂, -CN, -OE³, -SE⁴, -C(O)E⁵, - C(O)NE⁶E⁷, -COOE⁸, and -SO₃E⁹, wherein E¹ to E⁹ are independently selected from the group consisting of a hydrogen atom, an alkyl group, a cycloalkyl group, an alkenyl group, a cycloalkenyl group, an alkynyl group, an aryl group, and a heteroaryl group, and wherein R¹ to R⁵ may bind to each other to form one or more rings. Preferably, the remaining four of R¹ to R⁵ are independently selected from the group consisting of a hydrogen atom, an alkyl group, an alkenyl group, a halogen atom, -NE¹E², -NO₂, -CN, -OE³, -SE⁴, -C(O)E⁵, -C(O)NE⁶E⁷, -COOE⁸, and -SO₃E⁹, more preferably selected from the group consisting of a hydrogen atom, an alkyl group, a halogen atom, -NE¹E², and -OE³. Preferably, E¹ to E⁹ are independently selected from the group consisting of a hydrogen atom, an alkyl group, an alkenyl group, and an aryl group, more preferably E¹ to E⁹ are independently selected from a hydrogen atom and an alkyl group. Most preferably, each of the remaining four of R¹ to R⁵ is a hydrogen atom.

Preferably, the one of R¹ to R⁵ is one of R¹ to R³. Most preferably, the one of R¹ to R⁵ is R¹ and the remaining four are R² to R⁵.

R⁶, R⁷, R¹⁰, and R¹¹ are independently selected from the group consisting of a hydrogen atom, an alkyl group, a cycloalkyl group, an alkenyl group, a cycloalkenyl group, an alkynyl group, an aryl group, a heteroaryl group, a halogen atom, -NA¹A²,-NO₂, -CN, -OA³, -SA⁴, -C(O)A⁵, -C(O)NA⁶A⁷, -COOA⁸, and -SO₃A⁹, wherein A¹ to A⁹ are independently selected from the group consisting of a hydrogen atom, an alkyl group, a cycloalkyl group, an alkenyl group, a cycloalkenyl group, an alkynyl group, an aryl group, and a heteroaryl group, and wherein R⁶ and R⁷ and/or R¹⁰ and R¹¹ may bind to each other to form one or more rings. Preferably, R⁶, R⁷, R¹⁰, and R¹¹ are independently selected from the group consisting of a hydrogen atom, an alkyl group, an alkenyl group, an aryl group, a heteroaryl group, a halogen atom, -NA¹A², -OA³,-C(O)A⁵, -C(O)NA⁶A⁷, and -COOA⁸, more preferably R⁶, R⁷, R¹⁰, and R¹¹ are independently selected from the group consisting of a hydrogen atom, an alkyl group, an aryl group, a halogen atom, -NA¹A², -C(O)NA⁶A⁷, and -OA³, and more preferably R⁶, R⁷, R¹⁰, and R¹¹ are independently selected from the group consisting of a hydrogen atom, an alkyl group, and -C(O)NA⁶A⁷. Preferably, A¹ to A⁹ are independently selected from the group consisting of a hydrogen atom, an alkyl group, an aryl group, and a heteroaryl group, more preferably A¹ to A⁹ are independently selected from a hydrogen atom, an alkyl group, and an aryl group. The ring(s) potentially formed by R⁶ and R⁷ and/or R¹⁰ and R¹¹ may contain heteroatoms, such as O, S, and N, preferably N, to which further substituted or unsubstituted alkyl groups, alkenyl groups, aryl groups, and heteroaryl groups may be bonded. For example, R⁶ and R⁷ and/or R¹⁰ and R¹¹, particularly R¹⁰ and R¹¹, may bond to each other to form a piperidine ring, which may be further substituted, e.g. by an aralkyl group, such as a benzyl group. In case R¹⁰ and R¹¹ bond to each other to form one or more rings, R¹² is preferably a hydrogen atom. A respective preferred group CR¹⁰R¹¹R¹² is depicted in the following general Formula (4).

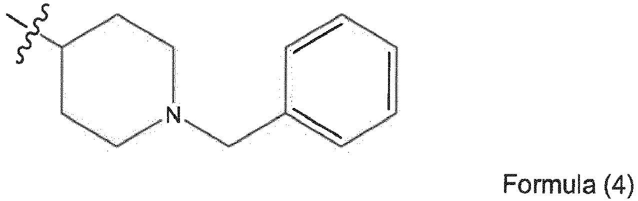

Most preferably, each of R⁶, R⁷, R¹⁰, and R¹¹ is a hydrogen atom.

According to the present invention, R⁸ and R⁹ are independently selected from the group consisting of a hydrogen atom, an alkyl group, a cycloalkyl group, an alkenyl group, a cycloalkenyl group, an alkynyl group, an aryl group, and a heteroaryl group. Preferably, R⁸ and R⁹ are independently selected from the group consisting of a hydrogen atom, an alkyl group, a cycloalkyl group, and an aryl group, more preferably from a hydrogen atom and an alkyl group. Most preferably, each of R⁸ and R⁹ is a hydrogen atom.

R¹² is selected from the group consisting of a hydrogen atom, an alkyl group, preferably having 1 to 9 carbon atoms, more preferably 1 to 6 carbon atoms, more preferably 1 to 5 carbon atoms, a cycloalkyl group, an alkenyl group, preferably having 2 to 11 carbon atoms, more preferably 3 to 10 carbon atoms, more preferably 4 to 9 carbon atoms, a cycloalkenyl group, an alkynyl group, preferably having 2 to 11 carbon atoms, more preferably 2 to 10 carbon atoms, more preferably 2 to 9 carbon atoms, an aryl group, preferably consisting of 1 to 3 rings, more preferably consisting of 1 or 2 rings, more preferably consisting of 1 ring, and a heteroaryl group, preferably consisting of 1 to 3 rings, more preferably consisting of 1 or 2 rings, more preferably consisting of 1 ring having heteroatoms. Preferably, R¹² is selected from the group consisting of an alkyl group, preferably having 1 to 9 carbon atoms, more preferably 1 to 6 carbon atoms, more preferably 1 to 5 carbon atoms, an alkenyl group, preferably having 2 to 11 carbon atoms, more preferably 3 to 10 carbon atoms, more preferably 4 to 9 carbon atoms, an aryl group, preferably consisting of 1 to 3 rings, more preferably consisting of 1 or 2 rings, more preferably consisting of 1 ring, and a heteroaryl group, preferably consisting of 1 to 3 rings, more preferably consisting of 1 or 2 rings, more preferably consisting of 1 ring having heteroatoms. More preferably, R¹² is selected from the group consisting of an alkyl group, preferably having 1 to 9 carbon atoms, more preferably 1 to 6 carbon atoms, more preferably 1 to 5 carbon atoms, an alkenyl group, preferably having 2 to 11 carbon atoms, more preferably 3 to 10 carbon atoms, more preferably 4 to 9 carbon atoms, and an aryl group, preferably consisting of 1 to 3 rings, more preferably consisting of 1 or 2 rings, more preferably consisting of 1 ring. More preferably, R¹² is selected from a n-propyl group, an iso-propyl group, an iso-butyl group, a 2,5-dichlorophenyl group, a 3,5-bis(trifluoromethyl)phenyl group, and a group of the following general formulas (5) to (7).

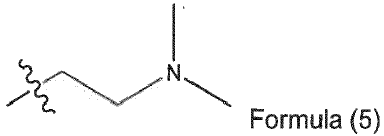

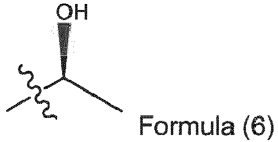

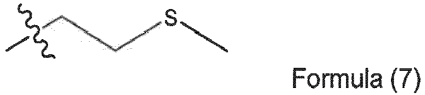

In said preferred embodiment, R¹⁰ is preferably a hydrogen atom and R¹¹ is selected from a hydrogen atom and -C(O)NA⁶A⁷, wherein A⁶ is a hydrogen atom and A⁷ is selected from an aryl group and a heteroaryl group. In case R¹² is selected from a n-propyl group, an iso-propyl group, a 2,5-dichlorophenyl group, a 3,5-bis(trifluoromethyl)phenyl group, and a group of the general formulas (5) and (6), R¹¹ is more preferably a hydrogen atom. In case R¹² is selected from an iso-butyl group and a group of the general formula (7), R¹¹ is more preferably a group of the following general formulas (8) and (9).

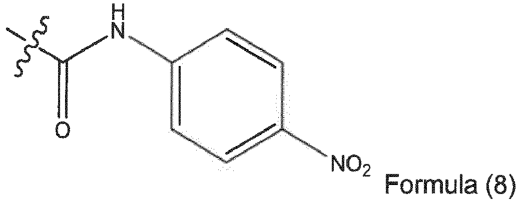

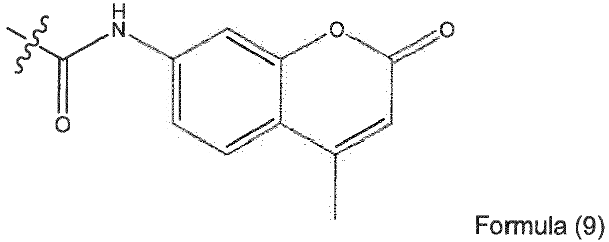

The compound according to the present invention may be a single enantiomer and/or a single diastereomer (if applicable) or a mixture of all possible isomers. For example, the CH group bonding to NR⁸, CR⁶R⁷(C₆R¹⁻⁵), and C(O)NR⁹CR¹⁰⁻¹² may be the (S) isomer or the (R) isomer or a mixture of both and in case R¹⁰ to R¹² differ from each other the CR¹⁰⁻¹² group may be the (S) isomer or the (R) isomer or a mixture of both. Preferably, the compound according to the present invention is the (S) isomer at the CH group bonding to NR⁸, CR⁶R⁷(C₆R¹⁻⁵), and C(O)NR⁹CR¹⁰⁻¹² and at the CR¹⁰⁻¹² group, if applicable.

More preferably, the compound of the general formula (1) is selected from the compounds of the following formulas (10) to (18), most preferably from the compounds of the following formulas (10) to (15).

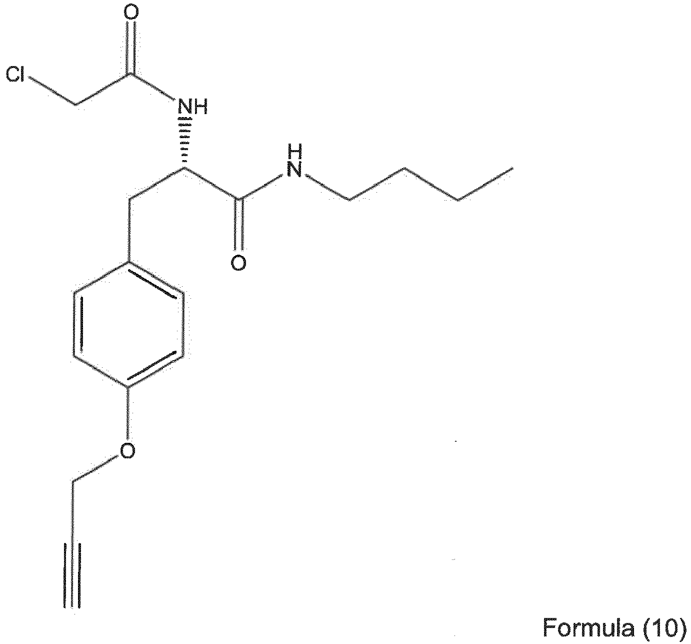

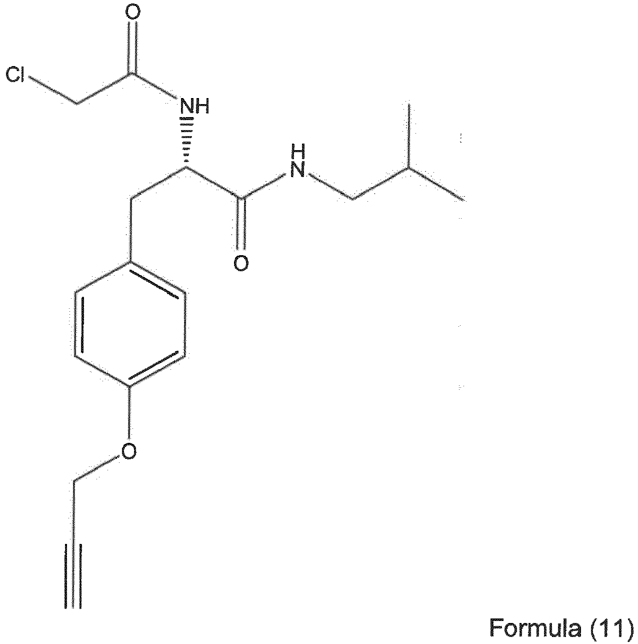

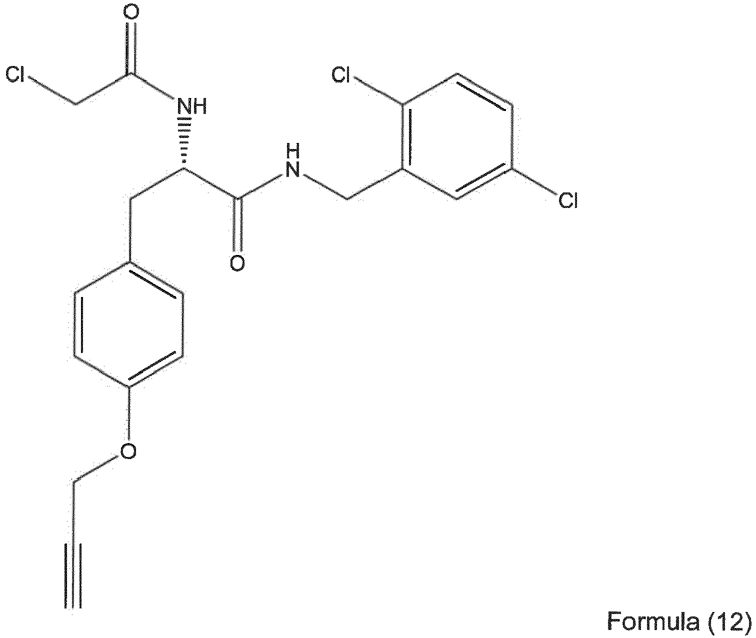

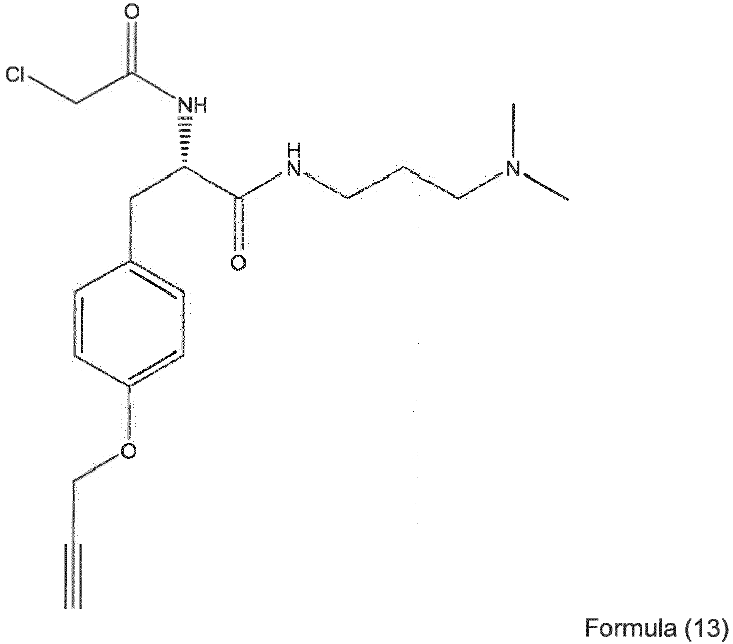

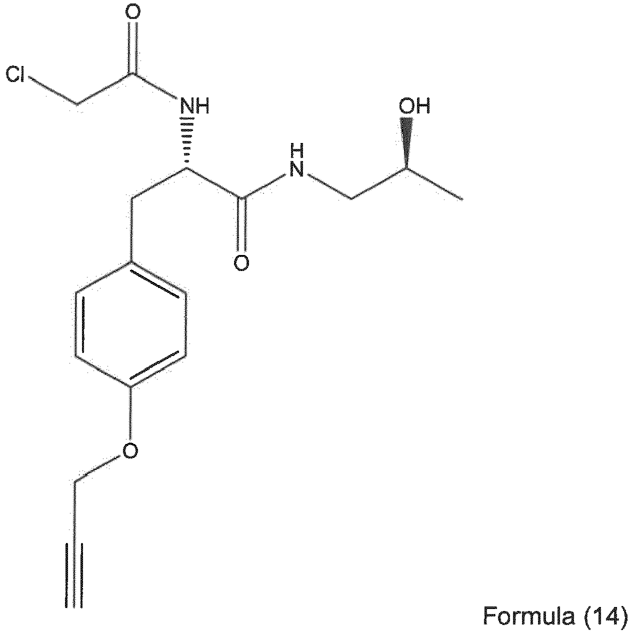

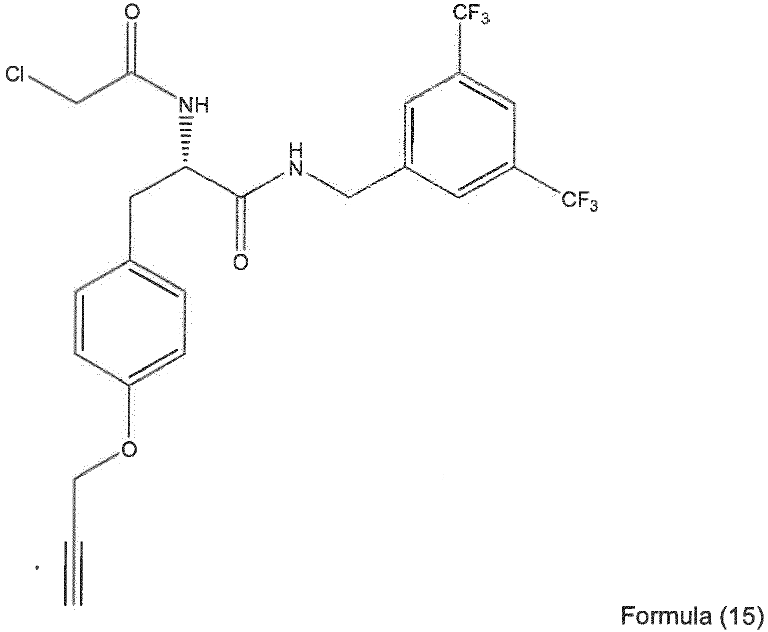

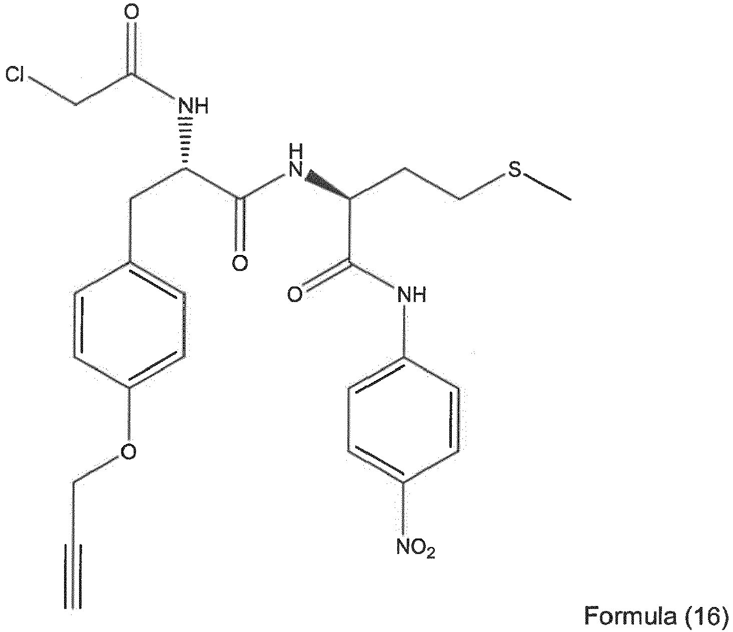

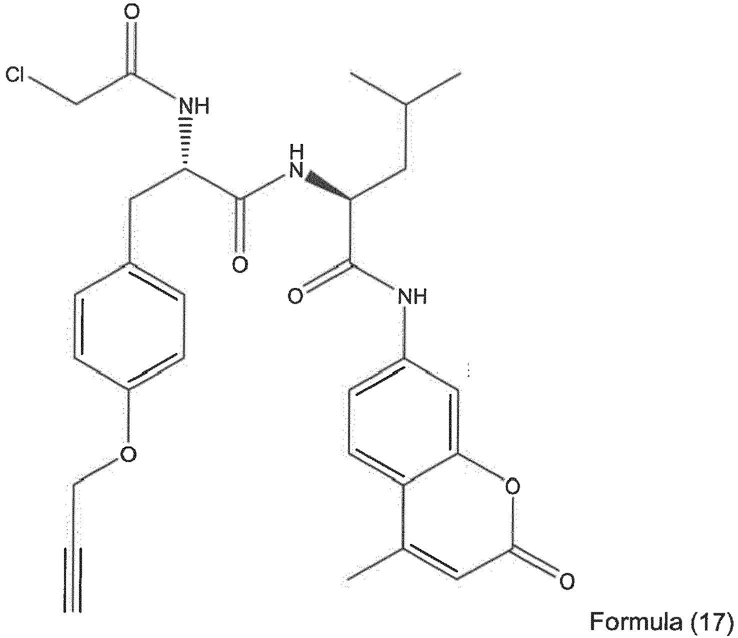

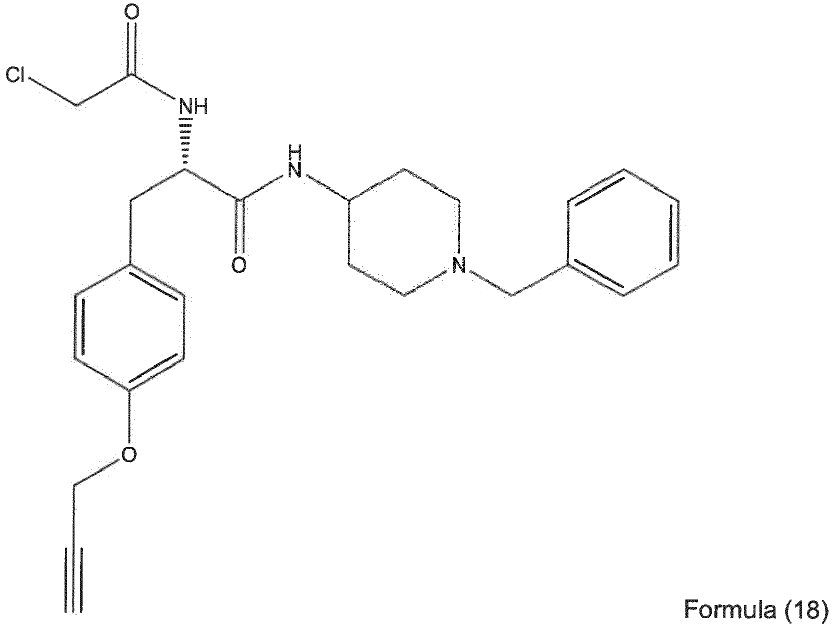

The compound according to the present invention may be the compound represented by the general Formula (1) as described above or a pharmaceutically acceptable salt thereof. In case the compound of the present invention is a pharmaceutically acceptable salt of the compound according to general Formula (1), the salt can be formed with inorganic or organic acids or bases. Examples of pharmaceutically acceptable salts comprise, without limitation, non-toxic inorganic or organic salts such as acetate derived from acetic acid, aconitate derived from aconitic acid, ascorbate derived from ascorbic acid, benzoate derived from benzoic acid, cinnamate derived from cinnamic acid, citrate derived from citric acid, embonate derived from embonic acid, enantate derived from heptanoic acid, formiate derived from formic acid, fumarate derived from fumaric acid, glutamate derived from glutamic acid, glycolate derived from glycolic acid, chloride derived from hydrochloric acid, bromide derived from hydrobromic acid, lactate derived from lactic acid, maleate derived from maleic acid, malonate derived from malonic acid, mandelate derived from mandelic acid, methanesulfonate derived from methanesulfonic acid, naphtaline-2-sulfonate derived from naphtaline-2-sulfonic acid, nitrate derived from nitric acid, perchlorate derived from perchloric acid, phosphate derived from phosphoric acid, phthalate derived from phthalic acid, salicylate derived from salicylic acid, sorbate derived from sorbic acid, stearate derived from stearic acid, succinate derived from succinic acid, sulphate derived from sulphuric acid, tartrate derived from tartaric acid, toluene-p-sulfonate derived from p-toluenesulfonic acid, sodium salts, potassium salts, magnesium salts, calcium salts, iron salts, zinc salts, aluminum salts, ammonium salts, and others. Such salts can be readily produced by methods known to a person skilled in the art.

Other salts like oxalate derived from oxalic acid, which is not considered as pharmaceutically acceptable, can be appropriately used as intermediates for the production of the compound of the general Formula (1) or a pharmaceutically acceptable salt thereof or physiologically functional derivative or a stereoisomer thereof.

The compound according to the present invention can be used in the treatment and prevention of an infection or a condition in a subject. The subject is not particularly limited. For example, the subject may be a vertebrate, preferably a mammal, more preferably a human.

The conditions and infections, which can be treated with the compound according to the present invention are for example caused by a SARS coronavirus, such as SARS-CoV-2. For example, such conditions and infections include, but are not limited to, COVID-19, respiratory, organ-specific or systemic infections.

As described above, the compound according to the present invention can be used as an inhibitor of proteases PL^{pro} and/or 3CL^{pro} of a SARS coronavirus, such as SARS-CoV-2. Preferably, the compound according to the present invention binds to the active site of the proteases. Preferably, the compounds according to formulas (10), (11), (13), and (14) are used as an inhibitor of protease 3CL^{pro} of a SARS coronavirus, such as SARS-CoV-2. Preferably, the compounds according to formulas (10) to (12), and (15) are used as an inhibitor of protease PL^{pro} of a SARS coronavirus, such as SARS-CoV-2.

The compound according to the present invention may be administered by any administration route known in the art being suitable for delivering a medicament to a subject, preferably a mammal. The route of administration does not exhibit particular limitations and includes for example oral application, topical application, intravenous application and intraperitoneal application. The compound or medicament may be present in the form of e.g. ointment, powders, drops or transdermal patches, or as an oral or nasal spray.

The dosage of the compound according to the present application can vary within wide limits and is to be suited to the individual conditions in each individual case. For the above uses the appropriate dosage will vary depending on the mode of administration, the particular condition to be treated and the effect desired. In general, however, satisfactory results may be achieved at dosage rates of about 1 µg/kg/day to 100 mg/kg/day animal body weight preferably 5 µg/kg/day to 50 mg/kg/day. Suitable dosage rates for larger mammals, for example humans, are of the order of from about 1 mg to 4 g/day, conveniently administered once, in divided doses such as e.g. 2 to 4 times a day, or in sustained release form. Moreover, the compound for use according to the present application can be applied topically to a locally defined site of infection, including but not limited to the eye, lung, or skin. In these cases, different dosages may be applied directly to the site of infection ranging from 1 ng/application to 5 g/application, preferably 1 ng/application to 1 g/application, more preferably 1 ng/application to 100 mg/application. Applications may vary from a single dose application or one application per day or one application every second day, to several applications per day such as two, three, four or five applications/day.

In another aspect, the present invention relates to a pharmaceutical composition comprising the compound according to the present invention or a pharmaceutically acceptable salt thereof in a pharmaceutically active amount, and optionally a pharmaceutically acceptable carrier, excipient or diluent. The above statements and definitions analogously apply to this aspect of the present invention. The compound of the present invention can be administered *per se* or in the form of pharmaceutical preparations.

The term "medicament" as used herein relates to any pharmaceutical composition comprising at least the compound according to the present invention in a pharmaceutically active amount.

The concentration of the compound of the present invention in the pharmaceutical composition of the present invention is not particularly limited. Preferably, the concentration of the compound of the present invention in the pharmaceutical composition is from 0.1 µM to 5 M, more preferably from 5 µM to 5 M, and most preferably from 10 µM to 100 mM.

In another aspect, the present invention relates to a method for producing a compound represented by the general formula (1), wherein the method comprises reacting a compound represented by the following general formula (2) 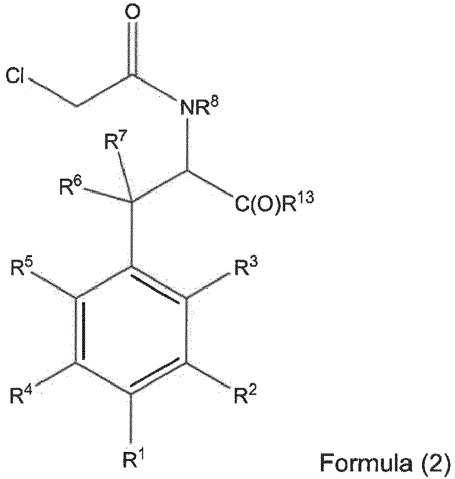 with a compound represented by the following general formula (3) 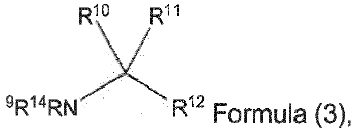
wherein R¹ to R¹² are defined as for the compound of the general formula (1);
R¹³ is selected from the group consisting of a pentafluorophenyloxy group, a tetrafluorophenyloxy group, a trifluorophenyloxy group, a difluorophenyloxy group, a fluorophenyloxy group, a phenyloxy group, a pentafluorophenylthio group, a tetrafluorophenylthio group, a trifluorophenylthio group, a difluorophenylthio group, a fluorophenylthio group, a phenylthio group, a trinitrophenyloxy group, a dinitrophenyloxy group, a nitrophenyloxy group, a *N*-succinimidyloxy group, and a benzotriazolyloxy group; and
R¹⁴ is selected from the group consisting of a hydrogen atom, an alkyl group, a cycloalkyl group, an alkenyl group, a cycloalkenyl group, an alkynyl group, an aryl group, and a heteroaryl group. The above statements and definitions analogously apply to this aspect of the present invention.

Preferably, R¹³ is selected from the group consisting of a pentafluorophenyloxy group, a tetrafluorophenyloxy group, a nitrophenyloxy group, a *N*-succinimidyloxy group, and a benzotriazolyloxy group, more preferably from the group consisting of a pentafluorophenyloxy group, and a *N*-succinimidyloxy group. Most preferably, R¹³ is a pentafluorophenyloxy group.

According to the present invention, R¹⁴ is selected from the group consisting of a hydrogen atom, an alkyl group, a cycloalkyl group, an alkenyl group, a cycloalkenyl group, an alkynyl group, an aryl group, and a heteroaryl group. Preferably, R¹⁴ is selected from the group consisting of a hydrogen atom, an alkyl group, a cycloalkyl group, and an aryl group, more preferably from a hydrogen atom and an alkyl group. Most preferably, R¹⁴ is a hydrogen atom.

Most preferably, the compound of the general formula (2) is the compound of the following formula (19).

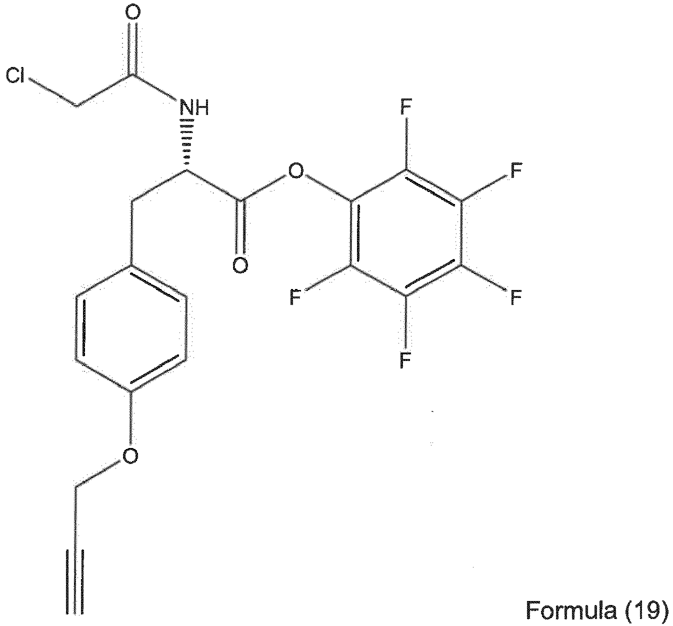

Preferably, the compound represented by the general formula (2) is reacted with the compound represented by the general formula (3) in the presence of a solvent. The applied solvent is not particularly limited. For example, the solvent may be selected from one or more of DMSO, butyl acetate, *N*,*N*'-dimethylformamide, N-methylpyrrolidinone dichloromethane, acetone, acetonitrile, ethyl acetate, ethyl propionate, ethyl butyrate, propyl acetate, propyl propionate, propyl butyrate, diethylether, dioxane, nitromethane, tetrahydrofuran, triethylamine, *N,N-*diisopropylethylamine, and pyridine. Preferably, the solvent is selected from one or more of DMSO, butyl acetate, ethyl propionate, ethyl butyrate, propyl acetate, propyl propionate, propyl butyrate, and pyridine. More preferably, the solvent is selected from one or more of DMSO, butyl acetate, and pyridine.

The concentrations of the compounds represented by the general formulas (2) and (3) are not particularly limited. Preferably, the concentrations of the compounds represented by the general formulas (2) and (3) are 0.001 mM to 1 M and 0.001 mM to 1 M, respectively, more preferably 0.1 mM to 100 mM and 0.1 mM to 100 mM, respectively, most preferably 0.5 mM to 5 mM and 0.5 mM to 5 mM, respectively. Preferably, the compound represented by the general formula (2) is reacted with the compound represented by the general formula (3) in the presence of a base. For example, the base may be selected from pyridine, triethylamine, 1,8-diazabicycloundec-7-ene, 2,6-di-*tert*-butylpyridine, and *N,N*-diisopropylethylamine. Preferably, the base is selected from pyridine, triethylamine, and *N,N-*diisopropylethylamine. More preferably, the base is pyridine.

Moreover, the temperature at which the compound represented by the general formula (2) is reacted with the compound represented by the general formula (3) is not particularly limited. For example, the temperature may be at least 0°C, preferably at least 10°C, and more preferably at least 15°C. The upper limit of the temperature is not particularly limited but may depend on the reactants and solvents used. For example, the upper limit of the temperature may be 90°C, preferably 60°C, and more preferably 30°C.

Furthermore, the duration for which the compound represented by the general formula (2) is reacted with the compound represented by the general formula (3) is not particularly limited. For example, the duration may be from 30 s to 4 d, preferably from 5 min to 1 d, more preferably from 20 min to 12 h, and more preferably from 40 min to 2 h.

The step of reacting the compound represented by the general formula (2) with the compound represented by the general formula (3) may be carried out in the presence of air or in the presence of an inert and/or dry atmosphere. Preferably, the step of reacting the compound represented by the general formula (2) with the compound represented by the general formula (3) is carried out in the presence of an inert and/or dry atmosphere. For example, the step of reacting the compound represented by the general formula (2) with the compound represented by the general formula (3) can be carried out under nitrogen atmosphere or argon atmosphere, preferably under argon atmosphere.

In potential subsequent steps, the compound represented by the general formula (1) obtained by reacting the compound represented by the general formula (2) with the compound represented by the general formula (3) may be purified and isolated by various methods known in the art. For example, unreacted starting material, potential converted base, and/or potential side-products may be removed by filtration, distillation, subsequent reactions or column chromatography from the reaction mixture. For example, the reaction mixture may be combined with (aminomethyl)polystyrene beads, preferably (aminomethyl)polystyrene beads (70-90 mesh), preferably in a new reaction vessel, for example for 15 min before subsequent isolation steps.

With the production method according to the present invention, it is preferably possible to generate a large library of compounds according to the present invention. The production method according to the present invention can for example be applied together with a screening method, which involves producing a large variety of compound according to the present invention and analyzing the inhibitory effects of said compounds with a target protein.

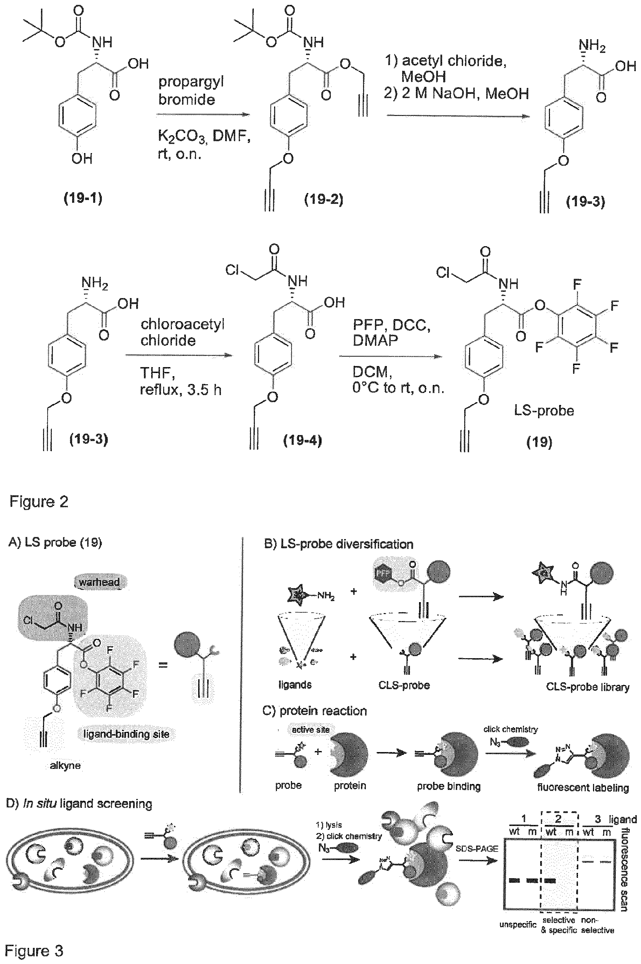

The compound according to general formula (2) can be regarded as a chemical probe which preferably targets the nucleophilic active site cysteine of a target protease PL^{pro} and/or 3CL^{pro}. The synthesis of one possible compound according to general formula (2) (LS-probe) is given in Fig. 2. The compound according to general formula (2) consists of a protein-reactive "warhead" and a ligand-binding site for the rapid screening of ligands/combinatorial probes (Fig. 3A). The LS-probe is reacted with various ligands to produce diversity. Unreacted probe can e.g. be removed using amine-functionalized polystyrene beads.

The target protein can then be exposed to the generated compound of the general formula (1) (ligand-loaded LS probe) (Fig. 3B) followed by e.g. click chemistry to append a fluorescent tetramethylrhodamine (TAMRA) tag (Fig. 3C). *In situ* reaction of the probe with overproduced PL^{pro} and/or 3CL^{pro}, e.g. obtained by heterologous expression in *E*. *coli.,* or in eukaryotic cells, allows to screen for cell permeable probes and active site mutant (Cys to Ala) allows to assess the specificity of a probe.

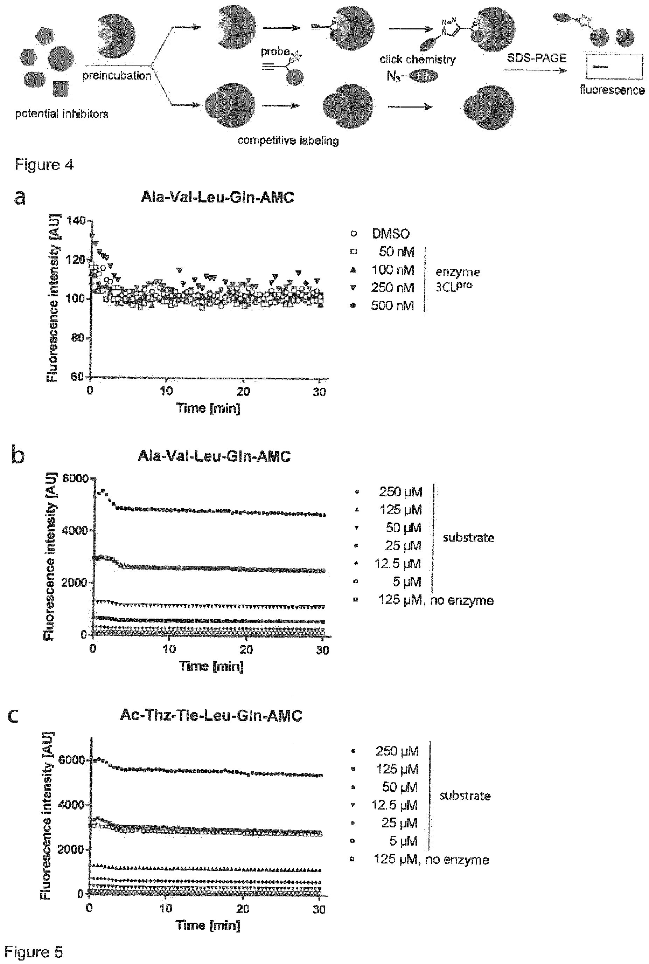

Specific and selective probes can be used for competitive profiling of enzyme inhibitors. This is e.g. performed by preincubation of PL^{pro}/3CL^{pro} expressing cells with potential inhibitors followed by competitive labeling of unbound enzyme with the probe. After e.g. cell lysis and click chemistry, potent inhibitors are identified by lack of fluorescence labeling on SDS-PAGE gels (Fig. 4).

In a further aspect, the present invention relates to a screening method comprising a contacting step (a) of bringing at least one compound represented by the following general formula (1) or a salt thereof in contact with (wild type) protease(s) PL^{pro} and/or 3CL^{pro} of a SARS coronavirus, wherein R¹ to R¹² are as defined above. The above statements and definitions analogously apply to this aspect of the present invention.

As outlined above, the compound according to the present invention can be analyzed with respect to its inhibitory effect and can be used for competitive profiling of enzyme inhibitors.

In a preferred embodiment, the screening method comprises a further contacting step (a2) of bringing the at least one compound represented by the general formula (1) or a salt thereof in contact with modified protease(s) PL^{pro} and/or 3CL^{pro} of a SARS coronavirus, which is/are modified in the active site of the wild type protease(s) PL^{pro} and/or 3CL^{pro} of a SARS coronavirus. The modification is such that the compound represented by the general formula (1) or a salt thereof does not covalently bond to the active site. For example, cysteine in the active sites of the proteases PL^{pro} and/or 3CL^{pro}, to which the compound according to the general formula (1) preferably bonds is exchanged to alanine. The steps (a) and (a2) can be carried out separately, such as in separate environments.

Preferably, in the screening method according to present invention, the contacting step (a), and the optional contacting step (a2), is/are carried out *in vitro.* The contacting step(s) can be carried out with isolated-protease(s) or can be carried out in one or more cells expressing the protease(s). Preferably, the contacting step (a), and the optional contacting step (a2), is/are carried out in one or more cells, such as *E*. *coli.* overproducing PL^{pro} and/or 3CL^{pro} by heterologous expression.

In one embodiment, the screening method according to the present invention further comprises, after the contacting step (a), a step (b) of adding a compound selected from the group consisting of a fluorophore-azide, a fluorophore-alkyne, a biotin-azide, or a biotin-alkyne. As outlined above, in the compound according to the present invention, one of R¹ to R⁵ contains an azide group, an alkynyl group, a biotin group, or a fluorophoric group. In case the one of R¹ to R⁵ does not already contain a biotin group or a fluorophoric group, such a group can be introduced via the azide group or the alkynyl group e.g. by click chemistry. For this purpose, for example a compound according to the present invention wherein one of R¹ to R⁵ contains an azide group is reacted with an alkyne bearing a biotin group or a fluorophoric group and vice versa. Further reagents used for alkyne-azide click chemistry, Staudinger ligation or other bioorthogonal reactions, which can further be added, are known in the art. In case the screening method is carried out in one or more cells, cell lysis is preferably carried out between the contacting step(s) and the step (b).

Preferably, the screening method according to the present invention further comprises an analyzing step (c) of analyzing as to whether the compound according to the present invention reacted with the protease(s). Suitable methods for carrying out such analysis are known in the art. For example, the samples (optionally after cell lysis) can be subjected to SDS-PAGE and absence and/or presence of fluorescence on the gels can be observed. In addition, antibody-based methods for detecting the probe are applicable. As alternative to SDS-PAGE, Capillary Gel Electrophoresis (CGE) can be used for signal detection.

In one embodiment, the screening method according to the present invention comprises a further contacting step (a0) of bringing the (wild type) protease PL^{pro} and/or 3CL^{pro} of a SARS coronavirus in contact with at least one compound to be screened before the contacting step (a) with the compound according to the general formula (1). As described above, the compound according to the present invention can be used as a probe for profiling the activity state of the protease(s) PL^{pro} and/or 3CL^{pro} of a SARS coronavirus, such as SARS-CoV-2. Thus, when first contacting a compound to be screened with the protease(s) of a SARS coronavirus of interest and later contacting with the compound according to the present invention competitive profiling of enzyme inhibitors can be achieved (cf. Figure 4). When carrying out the step (c), potent inhibitors can be identified by lack of fluorescence labeling e.g. on SDS-PAGE gels, or any antibody-based detection method using for example horseradish peroxidate (HRP)-conjugated antibodies, or surface plasmon resonance (SPR) with immobilization using a biotin tag. Preferably, a compound to be screened leads to lack of fluorescence labeling for one of PL^{pro} and 3CL^{pro}, more preferably for both of PL^{pro} and 3CL^{pro}. Suitable methods for screening enzyme inhibitors comprise competitive labelling experiments using pre-treatment of a protein sample with potential inhibitors followed by probe labelling and fluorescent SDS-PAGE analysis, Capillary Gel Electrophoresis (CGE) or analysis by a change in fluorescence polarization upon target binding with purified protein, cell lysates or inside live cells.

In a further aspect, the present invention relates to use of a rosmarinic acid derivative, a salvianolic acid derivative, a phenethyl isothiocyanate derivative, or a curcumin derivative as an inhibitor of proteases PL^{pro} and/or 3CL^{pro} of a SARS coronavirus, such as SARS-CoV-2. Moreover, in a further aspect, the present invention relates to a rosmarinic acid derivative, a salvianolic acid derivative, a phenethyl isothiocyanate derivative, or a curcumin derivative for use in the treatment or prevention of an infection or condition caused by a SARS coronavirus, such as SARS-CoV-2. The above statements and definitions analogously apply to this aspect of the present invention. Preferably, a rosmarinic acid derivative or a salvianolic acid derivative is used as an inhibitor of protease 3CL^{pro}. A phenethyl isothiocyanate derivative or a curcumin derivative is preferably used as an inhibitor of protease PL^{pro}. The term rosmarinic acid derivative for example relates to rosmarinic acid, isorinic acid, and caffeoyl phenylacetate. Preferably, the rosmarinic acid derivative is rosmarinic acid. The term salvianolic acid derivative for example relates to salvianolic acid A, salvianolic acid B, salvianolic acid C, salvianolic acid D, and lithospermic acid. Preferably, the salvianolic acid derivative is salvianolic acid A or salvianolic acid B, more preferably salvianolic acid A. The term phenethyl isothiocyanate derivative for example relates to phenethyl isothiocyanate, benzyl isothiocyanate, phenyl isothiocyanate, and allyl isothiocyanate. Preferably, the phenethyl isothiocyanate derivative is phenethyl isothiocyanate. The term curcumin derivative for example relates to curcumin, demethoxycurcumin, and bisdemethoxycurcumin. Preferably, the curcumin derivative is curcumin.

The figures show:
Fig. 1: Genome architecture of SARS-CoV-2. Genes encoding structural proteins are highlighted in black. The cleavage sites of PL^{pro} and 3CL^{pro} in the polyprotein are marked with arrows.
Fig. 2: Synthesis of one compound of general formula (2) (LS-probe).
Fig. 3: Method of ligand selection and screening using a compound according to general formula (2) (LS-probe). Wt = wild type; m = mutant.
Fig. 4: Method of competitive inhibitor profiling against PL^{pro} and 3CL^{pro}.
Fig. 5: Cleavage assays for purified 3CL^{pro} using fluorogenic (7-amino-4-methylcoumarin, AMC) peptide substrates. **a**, Using different enzyme concentrations from 50 nM to 500 nM. **b,** Using peptidic substrate concentrations between 5 µM and 250 µM. **c,** With a different peptidic substrate in the range from 5 µM to 250 µM. For **b** and c fluorescence intensities with and without enzyme overlap at 125 µM.
Fig. 6: Example for the ligand selection screening with live cell labelling of wild type (wt) and active site mutant (m) by the LS probe library at 20 µM. Representative data of three independent replicates.
Fig. 7: Quantification of wild type labelling intensities (wt) normalized to DMSO controls and specificity (wt/m) of LS probes at 20 µM. Specific probes exhibit a high wild type to mutant ratio (n=3). Criss-cross pattern: not determined.
Fig. 8: Concentration series of selected probes for labelling of 3CL^{pro} and PL^{pro}.
Fig. 9: Selectivity of compounds of formulas (13) and (15) at 20 µM in live cell labelling of proteases 3CL^{pro} and PL^{pro}, respectively, in the background of a native *E. coli* proteome. Flu: fluorescence gels; Coo: Coomassie stained gels. Representative gels of three independent replicates.
Fig. 10: Comparison of the *in situ* labelling of 3CL^{pro} of SARS-CoV-1 and SARS-CoV-2 in live heterologously expressing *E. coli* cells. The probes yield equal labelling intensity and specificity for the two 3CL^{pro} homologues.
Fig. 11: Labelling of 3CL^{pro} with compound of formula (13) and PL^{pro} with compound of formula (15) in the background of the native proteome of HepG2 cell lysates at 20 µM probe concentration. Controls (Δ heat) give the unspecific background labelling of the heat denatured proteome. Representative results of three independent experiments.
Fig. 12: Half-maximal inhibitory concentrations (IC₅₀s) determined from curve fittings of quantitative competitive labelling experiments. **a**, With compounds M26 and X05 inhibiting labelling of 3CL^{pro} by compound of formula (13) (IC₅₀ = 12 µM for M26 = SalB; IC₅₀ = 43 µM for X05). **b**, With compounds M03 and X05 inhibiting labelling of PL^{pro} by compound of formula (15) (IC₅₀ = 26 µM for M03; IC₅₀ = 81 µM for X05).
Fig. 13: Inhibition of PL^{pro} determined by an enzyme activity assay using the fluorogenic substrate Arg-Leu-Arg-Gly-Gly-AMC. **a,** Inhibition of PL^{pro} activity by X05 (IC₅₀ = 44 µM). **b**, Inhibition of PL^{pro} activity by M03 (IC₅₀ = 10 µM). **c**, Inhibition of PL^{pro} activity by compound of formula (15) (IC₅₀ = 58 µM).
Fig. 14: Inhibition curves of 3CL^{pro} inhibitors determined by quantification of fluorescent labeling intensity by compound of formula (13) (n=3). Lith: lithospermic acid; Ros: rosmarinic acid; SalA: salvianolic acid A; SalC: salvianolic acid C; SalB: salvianolic acid B.
Fig. 15: Examples of fluorescent gels with competitive labelling of 3CL^{pro}.
Fig. 16: IC₅₀ values of the most active inhibitors quantified by competitive fluorescence labeling.
Fig. 17: Competitive labelling with concentration series of extracts of the roots of *Salvia miltiorrhiza.*

The present invention will be further illustrated in the following examples without being limited thereto.

### Experimental procedures:

### General

### Click reactions

Click chemistry was performed using 40 µL cell lysate, 2 µL of a 0.65 mM tetramethylrhodamine (TAMRA) azide stock in DMSO, 4 µL of a 1.66 µM TBTA (tris((1-benzyl-4-triazolyl)methyl)amine) stock in *ter*t-butanol/DMSO (8:2 v/v). To start the cycloaddition 2 µL of a freshly prepared 52 mM TCEP (tris(2-carboxyethyl)phosphine hydrochloride) stock in water and 2 µL of a 50 mM CuSO₄ stock solution in water were added. The samples were incubated for 1 h at room temperature before quenching with 50 µL 2x SDS loading buffer (63 mM Tris-HCI, 10 % (v/v) glycerol, 2 % (w/v) SDS, 0.0025 % (w/v) bromophenol blue, 10 % (v/v) β-mercaptoethanol; dissolved in water).

### SDS-PAGE and in-qel fluorescence scanning

Before performing SDS-PAGE the samples were incubated for 10 min at 95 °C and subsequently centrifuged down. SDS gels containing 10 % acrylamide and an aqueous solution of 37.5:1 acrylamide and *N,N'*-methylenbisacrylamide were used with a PeqLab system and run at 75 mA per gel. Visualization was done by in-gel fluorescence scanning of the tetramethylrhodamine (TAMRA) dye (Fusion-FX7). Equal protein content and separation in SDS-gels was confirmed by Coomassie staining (InstantBlue^{™}, expedeon).

### Example 1: Synthesis of an LS-Probe

One LS-probe of the formula (19) was synthesized according to the following procedure (cf. Figure 2).

### 2-tert-Butoxycarbonylamino-3-[4-(prop-2-ynyloxy)phenyl]-propionic acid propargyl ester (19-2)

The reaction was adapted from a protocol described in the literature (Polic, V. & Auclair, K. Allosteric Activation of Cytochrome P450 3A4 via Progesterone Bioconjugation. Bioconjug Chem 28, 885-889 (2017)). 6 g of (21.3 mmol, 1 eq) *N*-*tert*-Butoxycarbonyltyrosine (19-1) and 9 g of (56.1 mmol, 3 eq) K₂CO₃ were suspended in 30 mL dry DMF under N₂ flow. After stirring for 10 min at room temperature 7.9 mL (73.1 mmol, 3.5 eq) of an 80 % solution propargyl bromide in toluene was slowly added. The solution was left to react for 18 h at room temperature. 150 mL H₂O were used to quench the reaction. The mixture was extracted with diethyl ether, washed with distilled water and brine. The combined organic layers were dried over MgSO₄ before solvent evaporation *in vacuo.* The yellow oil was used in the next step without any further purification (7.3 g, 100 %).
¹H-NMR (CDCl₃, 400 MHz) δ (ppm): 1.42 (s, 9 H, (CH₃)₃C-), 2.51 (m, 2 H, - CO₂CH₂CCH, -PhOCH₂CCH), 3.07 (m, 2 H, -PhCH₂CH-), 4.59-4.93 (m,
1 H, -PhCH₂CH-), 4.64-4.80 (m, 2 H, -CO₂CH₂CCH, -PhOCH₂CCH), 6.90 (m, 2 H, aromat.), 7.09 (m, 2 H, aromat.)

### 2-Amino-3-[4-(prop-2-ynyloxy)phenyl]-propionic acid propargyl ester (19-2b)

The reaction was adapted from a protocol described in the literature (Polic, V. & Auclair, K. Allosteric Activation of Cytochrome P450 3A4 via Progesterone Bioconjugation. Bioconjug Chem 28, 885-889 (2017)). To 180 mL of MeOH on an ice bath, 21 mL (294 mmol, 15 eq) acetyl chloride were slowly added. The solution was left to stir for 10 min at 0 °C. 7 g of (19-2) were added and the solution was allowed to warm to room temperature. After 2 h the solvent was evaporated *in vacuo* to give the pure product as slightly brownish-white powder (4.1 g, 100 %).
¹H-NMR (D₂O, 400 MHz) δ (ppm): 2.98 (t, *J* = 2.4 Hz, 1 H, -OCH₂CCH), 3.30-3.09 (m, 2 H, -PhCH₂CH-), 3.99 (dd, 1 H, *J* = 7.8, *J* = 5.2 Hz, -PhCH₂CH-), 4.85 (d, 2 H, *J* = 2.4 Hz, -OCH₂CCH), 7.10 (m, 2 H, aromat.), 7.33 (m, 2 H, aromat.).

### O-Propargyl tyrosine (19-3)

The reaction was adapted from a protocol described in the literature (Polic, V. & Auclair, K. Allosteric Activation of Cytochrome P450 3A4 via Progesterone Bioconjugation. Bioconjug Chem 28, 885-889 (2017)). To a mixture of 30 mL MeOH and 42 mL 2 M NaOH 5.6 g (20 mmol, 1 eq) (19-2b) was added. The reaction was stirred for 17 h at room temperature. With concentrated HCI the pH of the mixture was adjusted to 7 and it was kept at 4 °C for 4 h. The precipitate was filtered off and dried *in vacuo* to give the pure product as light yellow powder (3.51 g, 17.1 mmol, 83 %).
¹H-NMR (D₂O, 400 MHz) δ (ppm) 2.98 (m, 1 H, -PhOCH₂CCH), 3.12 (dd, 1 H, *J* = 14.9, *J* = 8.1 Hz, -PhCH₂CH-), 3.27 (dd, 1 H, *J* = 14.3, *J* = 4.9 Hz, -PhCH₂CH-), 4.00 (td, 1 H, *J* = 13.1, *J* = 2.2 Hz, -PhCH₂CH-), 4.85 (t, 2 H, *J* = 1.7 Hz, -PhOCH₂CCH), 7.11 (m, 2 H, aromat.), 7.32 (m, 2 H, aromat.).

### 2-(2-Chloroacetamido)-3-(4-(prop-2-yn-1-yloxy)phenyl)propanoic acid (19-4)

The reaction was adapted from a protocol described in the literature (Beagle, L.K. et al. Efficient Synthesis of 2,5-Diketopiperazines by Staudinger-Mediated Cyclization. Synlett, 2337-2340 (2012)). 1.03 g (5 mmol) of (19-3) were suspended in 35 mL of dry THF under an N₂ flow. 0.6 mL (1.5 eq) of chloroacetyl chloride were added. The suspension was stirred at reflux for 3 hours. The reaction was extracted with ethyl acetate and washed with distilled water and brine. The combined organic layers were dried over MgSO₄ before solvent evaporation *in vacuo.* The yellow crystals were purified via column chromatography (DCM:MeOH=9:1) to give the pure product as light yellow crystals (1.19 g, 85 %).
¹H-NMR (CDCl₃, 400 MHz) δ (ppm): 2.52 (t, 1 H, *J* = 2.2 Hz, -OCH₂CCH), 3.09-3.23 (m, 2 H, -PhCH₂CH-), 4.05 (d, *J* = 2.0 Hz, 2 H, -OCH₂CCH), 4.68 (d, *J* = 2.5 Hz, 2 H, -CH-Cl₂), 4.86 (m, 1 H, -PhCH₂CH-), 6.94 (d, 2 H, *J* = 8.6 Hz, aromat.), 7.11 (d, *J* = 8.4 Hz, 2 H, aromat.).
ESI-HRMS: m/z = 296.0685 [M+H]⁺, calc. for C₁₄H₁₄ClNO₄ + H⁺ = 296.0684.

### Perfluorophenyl 2-(2-chloroacetamido)-3-(4-(prop-2-yn-1-yloxy)phenyl)propanoate (19, LS-probe)

The reaction was adapted from a protocol described in the literature (Liu, Y. et al. Building Nanowires from Micelles: Hierarchical Self-Assembly of Alternating Amphiphilic Glycopolypeptide Brushes with Pendants of High-Mannose Glycodendron and Oligophenylalanine. J Am Chem Soc 138, 12387-94 (2016)). 0.92 g (3.2 mmol) of (19-4) and 0.72 g (3.6 mmol) of pentafluorophenol were dissolved in 80 mL dry DCM under nitrogen. The solution was stirred on ice for 20 min before adding 40 mg of DMAP (0.32 mmol) and 0.74 g DCC (3.6 mmol). The suspension was stirred over night at room temperature. The reaction was quenched with 4 mL 3N HCI. The resulting solution was kept at 4 °C over night. The precipitate was filtered off over celite and washed with cold DCM. Afterwards the filtrate was washed with saturated NaHCO₃ solution and distilled water, dried over MgSO₄ before the solvent was evaporated *in vacuo.* The solid was purified by flash chromatography (ethyl acetate, hexane) to give 0.93 g (63 %) of the pure product as pale yellow crystals.
¹H-NMR (CDCl₃, 400 MHz) δ (ppm):, 2.51 (t, 1 H, *J* = 1.8 Hz, -OCH₂CCH), 3.11-3.23 (m, 2 H, -PhCH₂CH-), 4.05 (d, *J* = 4.5 Hz, 2 H, -OCH₂CCH), 4.68 (d, *J* = 2.1 Hz, 2 H, -CH-Cl₂), 4.87 (dt, *J* = 6.32, *J* = 7.1 Hz, 1 H, -PhCH₂CH-), 6.94 (d, 2 H, *J* = 8.5 Hz, aromat.), 6.96 (s, 1 H, -NH-), 7.11 (d, *J* = 8.7 Hz, 2 H, aromat.).
¹³C-NMR (CDCl₃, 400 MHz) δ (ppm): 36.93 (PhCH₂CH-), 42.81 (-CH₂Br), 53.73 (-PhCH₂CH-), 56.33 (-OCH₂CCH), 76.09 (-OCH₂CCH), 77.96 (-OCH₂CCH), 115.74 (aromat.), 130.83 (aromat.), 157.48 (aromat.), 166.60 (-COO-), 174.96 (-NCO-). ¹⁹F-NMR (CDCl₃, 400 MHz) δ (ppm): -152.67 (d, 2F, *ortho*), -157.57 (t, 1F, *para*), *-* 162.42 (t, 2F, *meta*).

### Example 2: Recombinant proteins

Both 3CL^{pro} and PL^{pro} likely liberate themselves from the polyprotein by cleaving their respective *N-* and *C*-terminal sequences as described for the homologous sequences of SARS-CoV-1. Hereby dimerization and maturation of 3CL^{pro} by *N*-terminal self-cleavage is required for full activation of the protease and the ability of trans-processing of other non-structural proteins. Dimeric crystal structures of mature 3CL^{pro} show that the *N*-terminus of one 3CL^{pro} monomer is in close proximity to the active site pocket of the other monomer and the *N*-terminus is cleaved between the short consensus sequence Leu-Gln and Ser already during expression. Molecular modelling of the flexible peptide sequence prior to cleavage indicated that the first amino acids before the cleavage site occupy a part of the active site pocket which is in agreement with mechanistic models of protease maturation. It was reasoned that inhibitors targeting the protease prior to full activation could be of great value for drug development against SARS-CoV-2. Thus, there was constructed a 3CL^{pro} version fused with a non-cleavable *N*-terminal Strep-tag II. Modelling predicted an identical behavior of the fusion peptide at the active site compared to the native sequence of wild type prior to cleavage. Codon optimized sequences of the 3CL^{pro} and PL^{pro} domains of nsp3 and nsp5 of SARS-CoV-2 were ultimately cloned into an IPTG inducible vector and heterologously expressed in *Escherichia coli.* Proteins were either purified via affinity chromatography or used *in situ* in the native cell of the expression system. Confirming the predictions regarding the *N*-terminal modification, the purified 3CL^{pro} version indeed was inactive in a protease assay using oligopeptide substrates linked to a fluorogenic 7-amino-4-methylcoumarin group (Fig. 5). This represented the unique opportunity to validate the utility of the LS-ABPP (LS-activity-based protein profiling) strategy against PL^{pro} and the pre-activation stage of 3CL^{pro} of SARS-CoV-2 and demonstrate its versatility for customizing probes to different targets. In order to discover probes with specificity for the active site of the proteases, there were also constructed the corresponding active site mutants Cys145Ala (3CL^{pro}) and Cys114Ala (PL^{pro}). The experimental details are given in the following.

### Plasmid preparation

The gene coding for the herein used proteins (3CL^{pro}, PL^{pro} and their mutants) were custom synthesized and constructed in a pET-51b(+) plasmid (GenScript, New Jersey) for protein expression in *E. coli* BL21 cells. The expression vector was IPTG inducible with ampicillin marker and *N*-terminal Strep-tag II with cloning site Kpnl-BamHI.

### Transformation of plasmids

For preparation of competent cells, 1 mL of an overnight culture of *E. coli* BL21 cells was inoculated in 25 mL of LB medium and kept at 37 °C and 180 rpm. At an OD₆₀₀ of 0.5 the cells were transferred into a 50 mL tarson tube and kept on ice for 20 min. Centrifugation at 6000 rpm and 4 °C for 10 min was performed. The supernatant was discarded and the cell pellet resuspended into 20 mL ice cold calcium chloride (50 mM). After an incubation time of 20 min on ice the cells were centrifuged at 6000 rpm for 10 min at 4 °C and the supernatant discarded. The cell pellet was again resuspended in 4.25 mL of calcium chloride solution (50 mM) as well as 0.75 mL of glycerol. Aliquots of 200 µL were made and directly put into liquid nitrogen before storing them at -80 °C.

An aliquot of competent *E. coli* BL21 cells was thawed on ice. 1 µL of the corresponding plasmid (50 ng/mL) was added to the cells and mixed by tabbing the tube three times. After incubation on ice for 30 min the cells were heat shocked at 42 °C for 45 sec. Afterwards the tube was placed on ice for 3 min. 900 µL LB media was added to the cells and they were kept at 37 °C for 1 h whilst shaking at 550 rpm. Centrifugation at 8000 rpm and 4 °C for 2 min was performed. The supernatant was discarded and the cell pellet resuspended. The cell suspension was streaked on ampicillin plates (100 µg/L), which were kept at 37 °C overnight. The next day colonies could be picked for overnight cultures which could be used to prepare glycerol stocks.

### Overproduction of proteins

Overnight cultures for cellular assays were prepared by taking a small amount of a bacterial cryo-stock (15 % glycerol, stored at -80 °C) and inoculating them in 5 mL LB in sterile 13 mL polypropylene tubes (Sarstedt, ref 62.515.028), supplemented with antibiotics as indicated and grown for 14-16 h at 37 °C (180 rpm). 1 mL of a corresponding overnight culture was inoculated in 50 mL LB medium containing 100 µL/mL ampicillin and kept at 37 °C and 180 rpm. At an OD₆₀₀ of 0.3 the protein expression was induced by adding 0.2 µg/mL of an IPTG solution (1 M). The cells were incubated at 37 °C and 180 rpm for 2 h before centrifugation at 4000 rpm for 20 min at 4 °C.

### Affinity-purification of proteins

For purifying the proteins overexpression was performed in a scale of 3 L as described. The cell culture was centrifuged down at 4000 rpm for 20 min at 4 °C. The supernatant was discarded and the cell pellet washed with 15 mL PBS before centrifuging at 4000 rpm for 20 min at 4 °C. The cells were lysed by ultrasound treatment (25 % amplitude, 0.5 s ON, 2.1 s OFF, 20 pulses, Branson Digital Sonifier). Afterwards samples were centrifuged for 1 h at 4000 rpm and 4 °C. The supernatant was transferred into a new 50 mL flask and put on ice before performing affinity purification via Strep-tag II on an ÄKTA start (GE Healthcare) using StrepTrap HP columns (GE Healthcare). Standard Bradford protocols were used to calculate the concentration of the protein fractions. 200 µL aliquots of 0.4 mg/mL protein were frozen in liquid nitrogen before storing them at -80 °C.

### Enterokinase digest of 3CL^{pro}

For cleavage of the N-terminal Strep-tag II, 2 µL enterokinase (0.3 mg/mL, Boehringer Mannheim) was added to 25 µL purified recombinant 3CL^{pro} (0.8 mg/mL) in phosphate-free buffer (50 mM Tris-HCI, 1 mM EDTA, pH = 7.3) and the mixture was incubated at 37 °C for 3.5 h.

### Example 3: LS-probe modification with ligands (Fig. 3B) and in vitro probe labelling

The LS probe was reacted individually with a selection of different amine containing ligands in microliter scale. Time-resolved ¹⁹F NMR spectra helped to optimize the reaction conditions and revealed complete conversion after 60 min even for the least reactive aromatic amines. Although a quantitative reaction was expected, potential residues of unreacted probe were removed using amino-functionalized polystyrene beads to prevent unspecific protein reactivity. Subsequently, the reaction mixture was lyophilized and dissolved in DMSO before direct application to protein labeling experiments. The LS-probe modification was conducted by the following general protocol and the compounds of the formulas (10) to (18) were successfully prepared by said protocol.

In a 1.5 mL micro reaction tube were added 2.5 µL of 5 M pyridine (in DMSO), 2.5 µL of a probe stock (1 mM in butyl acetate) as well as 2.5 µL of the corresponding amine containing ligand (1 mM in DMSO) to result in a final concentration of 50 µM in the cell suspension. The mixture was incubated for 1 h at room temperature. Quenching was performed by adding 50 µL butyl acetate to the reaction mixture and pipetting the entire solution into a fresh 1.5 mL micro reaction tube containing 5 mg of (aminomethyl)polystyrene beads (70-90 mesh, Sigma-Aldrich). After incubation for 15 min the supernatant was transferred into a fresh 1.5 mL micro reaction tube. The beads were washed with 50 µL of butyl acetate and the supernatants were combined. The pooled solution was dried at high vacuum. 2 µL of DMSO were used to dissolve the reacted probe. For dose down experiments the same procedure as for the reaction of a LS-probe and ligand was used. To get a dose down of a final concentration of 50 µM, 20 µM, 10 µM, 5 µM, 1 µM and 0.1 µM in 50 µL the respective amount of ligand and probe was used.

### In vitro probe labelling of proteins in lysates and purified protein (Fig. 3C)

The respective protein aliquot (0.4 mg/mL) was thawed on ice. Reaction of LS probe and ligands was performed as described. The reacted probe was dissolved in 2 µL DMSO and added with 8 µL PBS to 10 µL protein solution before incubation for 30 min at 400 rpm and 37 °C. After incubation Click Chemistry was performed.

### Example 4: In situ probe labelling of proteins in live E. coli cells (Fig. 3D) and in situ competitive profiling

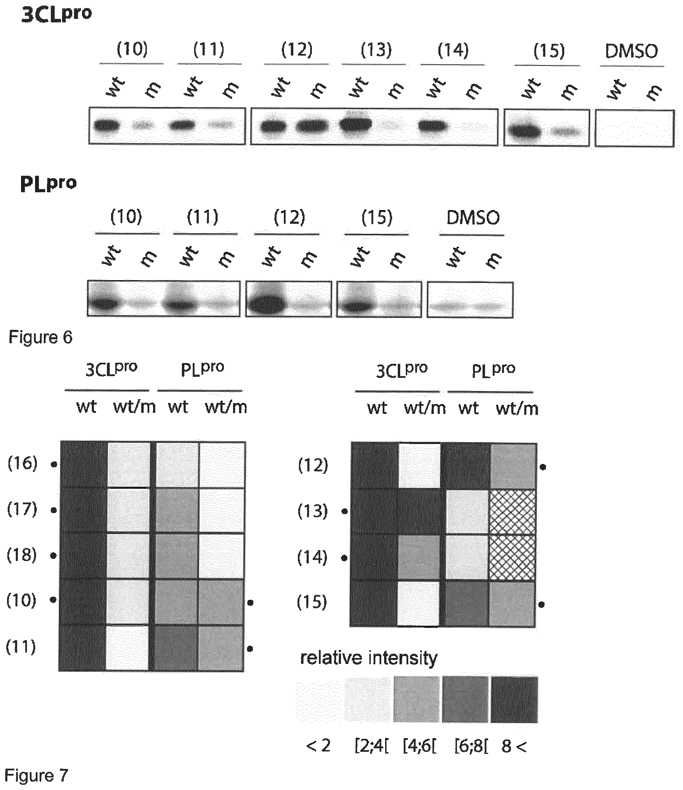

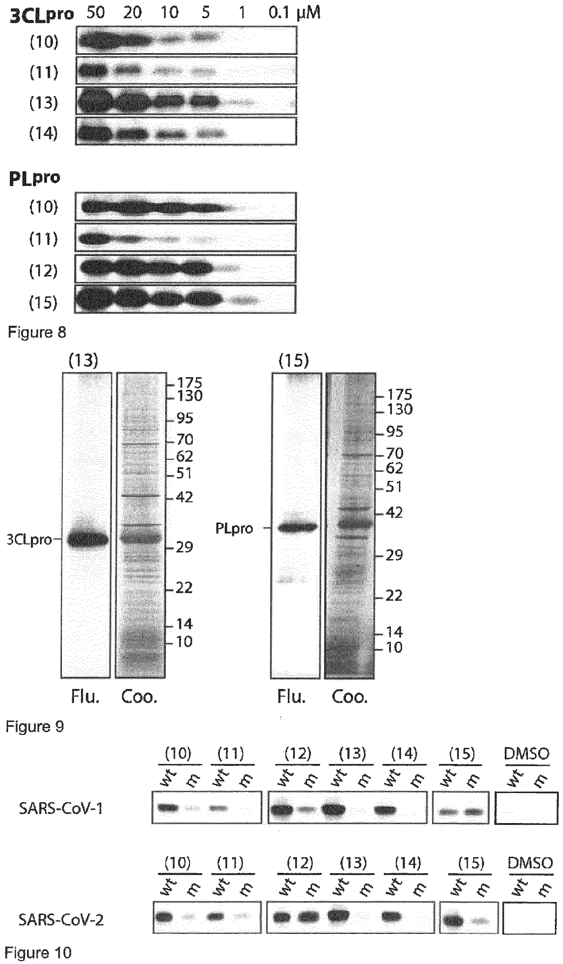

The probes obtained in Example 3 were screened *in situ* against 3CL^{pro} and PL^{pro} expressed in intact *E. coli* cells and their corresponding active site mutants Cys145Ala (3CL^{pro}) and Cys114Ala (PL^{pro}). (Fig. 3D). To this aim, the cells were incubated with 20 µM of the ligand modified probes obtained in Example 3 for one hour, followed by cell lysis and click chemistry with tetramethylrhodamine (TAMRA) azide to append a fluorescent reporter tag. After gel electrophoresis by SDS-PAGE, fluorescence imaging revealed probe labeling of the enzymes (Fig. 3D). The probes resulted in strongly labelled bands of 3CL^{pro} and PL^{pro}. The probes were further examined for their specificity by comparing labelling of wild type (wt) versus active site mutant (m) proteins (Fig. 6). Fluorescence intensities were quantified relative to the DMSO control and probes with a ratio wt/m > 2 were considered specific. The probes of Example 3 exhibited great specificity for labelling only the wild type but not mutant 3CL^{pro} and PL^{pro} (Fig. 7). Compounds (13) and (14) showed particular specificity for labelling of 3CL^{pro}. Compounds (10) to (12) and (15) were particular specific for PL^{pro}. It was thus possible to identify complementary probes for the two proteases. To estimate their sensitivity, there were performed labelling experiments with the most specific probes in concentration dependence. Strikingly, 3CL^{pro} was labelled by compound (13) and PL^{pro} by compounds (12) and (15) as the most sensitive probes at concentrations as low as 1 µM (Fig. 8). Interestingly, overproduced 3CL^{pro} and PL^{pro} were the only bands that compounds (13) and (15) labelled in live *E. coli* cells emphasizing the selectivity of the probes in the background of a native proteome (Fig. 9). The experiments were conducted according to the following protocol.

After overexpressing a protein in *E. coli,* for each sample 1 mL of the induced bacterial culture was transferred into a 1.5 mL Eppendorf tube. The cells were pelleted by centrifugation (4000 rpm, 7 min, 4 °C), washed with 50 µL PBS before re-suspending them in 48 µL PBS. The reacted and quenched probes, solved in 2 µL DMSO were added to the cell suspension before incubation at 400 rpm at 37 °C for 1 h. After incubation the cells were pelleted by centrifugation (4000 rpm, 5 min, 4 °C) washed with 50 µL PBS and resuspended in 50 µL PBS. The cell suspension was stored at - 80 °C before further processing. After thawing the samples, they were lysed by ultrasound treatment (10 % amplitude, 0.5 s ON, 1 s OFF, 10 pulses, Branson Digital Sonifier). The resulting lysates were used for click chemistry and SDS-PAGE. After fluorescence scanning, Coomassie staining was applied to compare protein concentrations in the gel and validate the experiments.

### In situ competitive profiling

The respective protein was overproduced in *E. coli* BL21 cells as described before. Reaction of LS probe and ligand was performed as described with 0.25 µL of LS-probe (1 mM), ligand (1 mM) and 5 M pyridine per sample. In the meantime, 1 µL of competitive molecule (10 mM) was incubated with 10 µL of the protein aliquot and 49 µL cell suspension for 1 h at 25 °C and 400 rpm. Each sample of the reacted probe was dissolved in 0.25 µL DMSO and added to the cell suspension before incubation for 1 h at 400 rpm and 37 °C. The cells were further treated like described for *in situ* labeling with LS-probes.

### Example 5: Comparing homologues of 3CL^{pro}

In order to verify that the LS-probe strategy also allows to label 3CL^{pro} of the closely related SARS-CoV-1, another member of the *Sarbecovirus* subgenus, there was also expressed its wild type and mutant sequences and performed LS probe labelling experiments *in situ* by the protocol given in Example 4. Labelling intensity and specificity for the active site of both 3CL^{pro} homologs were virtually identical for all tested compounds (cf. Fig. 10), demonstrating robustness even across different viral strains.

### Example 6: LS-probe labelling of virus proteases in background of HepG2 proteomes

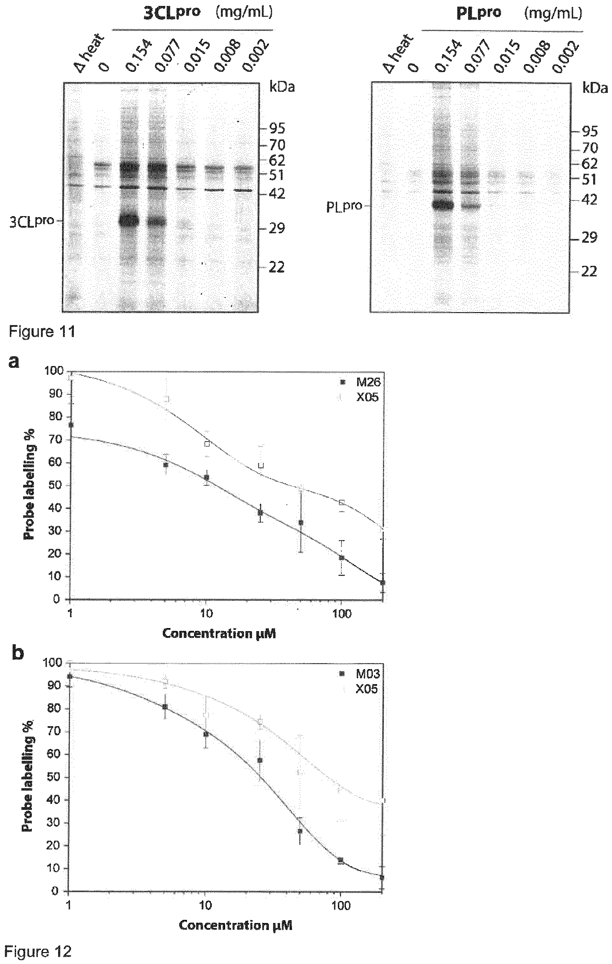

The potential application of the probes to label the proteases in the background of a native human proteome was also investigated. Lysates of human hepatocellular carcinoma (HepG2) cells were used and supplemented with different concentrations of purified 3CL^{pro} and PL^{pro}. Applying compound of formula (13) for 3CL^{pro} and compound of formula (15) for PL^{pro} at 20 µM followed by click chemistry resulted in the detection of both proteases as strong bands at protease concentrations down to 77 µg/mL (Fig. 11). Only relatively few additional off-target bands were labelled in HepG2 cell lysates suggesting that these probes could be employed to label and detect the activity of SARS-CoV-2 proteases in the background of a complex proteome. The following procedures were applied for the experiments.

Human hepatocellular carcinoma HepG2 (ATCC, HB-8065) were maintained in DMEM (4.5 g/L glucose, pyruvate) (Gibco), supplemented with 10 % fetal bovine serum (PAA Laboratories) and penicillin/streptomycin (25 µg/mL each) at 37 °C, 5 % CO₂ (Gibco). Cells were seeded in T75 flasks at a density of 60.000 cells/cm² and allowed to proliferate for 3 days. For the experiments, 2 x 10⁸ cells were harvested and lysed by sonication in PBS (3x 25% amplitude, 0.5 s ON, 2.1 s Off, 20 pulses).

The respective protein aliquot (0.4 mg/mL) as well as the HepG2 cell lysate aliquot were thawed on ice. Reaction of the LS-probe was performed as described before with a final concentration of the reacted probe of 20 µM. Samples for the protein dose down containing 10 µg, 5µg, 1 µg, 0.5 µg and 0.1 µg of protein were prepared with 40 µL of HepG2 cell lysate (1.5 mg/mL), the dissolved and reacted LS-probe and PBS to give a volume of 65 µL. Incubation of 30 min at 400 rpm and 37 °C was performed before click reaction.

### Example 7: Competitive screening for identifying enzyme inhibitors

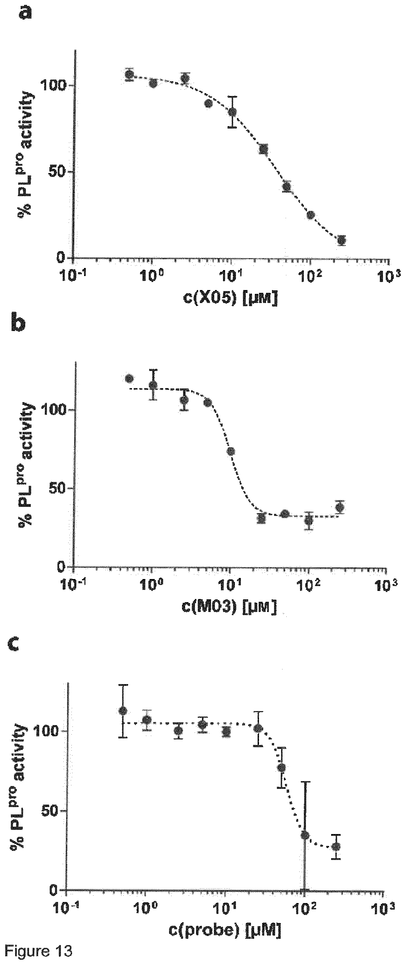

Since the probes exhibited sensitive and active site-specific labelling of PL^{pro} as well as of the pre-activation stage of 3CL^{pro}, their potential as chemical tools for the competitive screening of enzyme inhibitors was investigated. There were searched over 1000 structures of commercially available food grade additives, natural products, and protease inhibitors and manually selected 44 compounds with electrophilic motifs such as aldehydes, Michael acceptors, epoxides, and esters that could potentially react covalently with the nucleophilic active site cysteine of 3CL^{pro} and PL^{pro}. The electrophilic compound library was prepared in form of DMSO stocks and individually pre-incubated for an initial screening with the purified proteases followed by labelling with the compound of formula (13) for 3CL^{pro} and the compound of formula (15) for PL^{pro}. Successful inhibitors would block the active site and thereby prevent subsequent probe labelling which could be read out by lowered in-gel fluorescence. Indeed, at an initial concentration of 200 µM some compounds even abolished probe labelling. Then there was quantified the fluorescence signal relative to the control and compounds that resulted in more than 50% inhibition of competitive labelling were selected to investigate their dose response relationship. Half maximal inhibitory concentrations (IC₅₀s) were calculated from curve fittings of concentration-dependent quantitative competitive labelling. Phenethyl isothiocyanate, which is produced from its precursor gluconasturtiin by vegetables of the *Brassicaceae* family, scored against both proteases likely due to unspecific thiol reactivity of isothiocyanates, but only led to incomplete inhibition (X05, Fig. 12). In contrast, curcumin (M03) almost completely inhibited labelling of PL^{pro} with an IC₅₀ of 26 µM and was inactive against 3CL^{pro} (Fig. 12). However, curcumin is a well-known pan-assay interference (PAIN) compound and as such of no particular interest. Both X05 and M03 inhibited enzyme activity of PL^{pro} with a fluorogenic peptide substrate, confirming the validity of the competitive screening approach (Fig. 13).

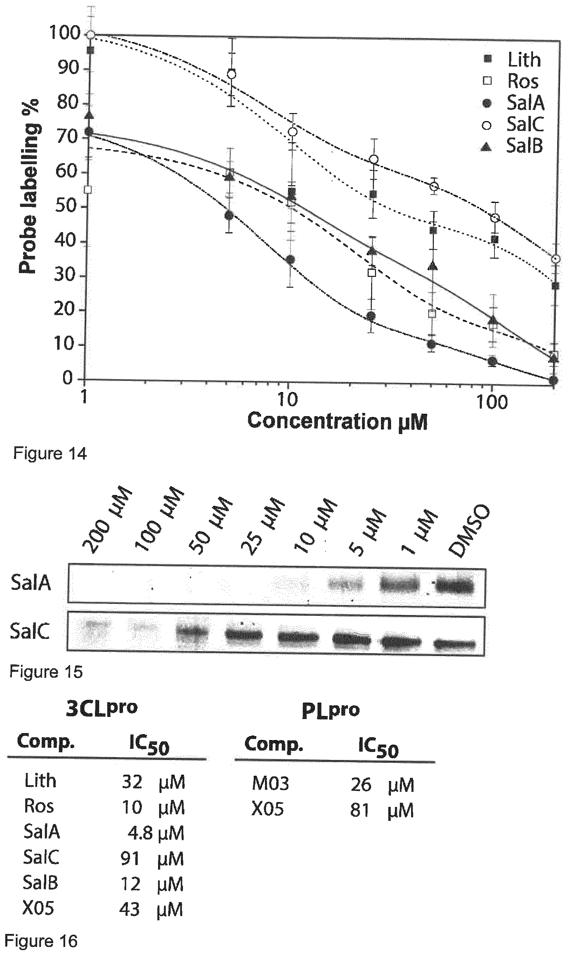

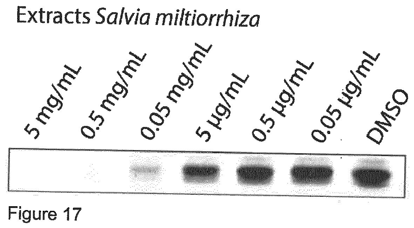

More interesting was the activity of salvianolic acid B (M26, SalB), which inhibited labelling of 3CL^{pro} with an IC₅₀ of 12 µM (Figs. 14 and 15). Thus, there was focussed on compounds with a closely related caffeic acid ester motif. All compounds showed a clear dose response behavior whereby rosmarinic acid (Ros) and salvianolic acid A (SalA) were the most active with IC₅₀ values of 10 µM and 4.8 µM, respectively. Interestingly, salvianolic acid C (SalC), which differs from SalA only by a hydroxyl group locked into a benzofuran ring, was considerably less active with an IC₅₀ of 91 µM (Figs. 15 and 16). Also, the closely related lithospermic acid (Lith) only exhibited an IC₅₀ of 32 µM. These results demonstrate a fine-tuned structure activity relationship of salvianolic acid derivatives for the inhibition of 3CL^{pro}. Since SalA and the other potent salvianolic acid derivatives are produced by the plant *Salvia miltiorrhiza* (red sage), there were prepared and tested extracts of its dried roots in a competitive labelling assay with compound of formula (13) against 3CL^{pro} and there was observed potent inhibition down to 1 mg/mL (Fig. 17). The following procedures were used for the above experiments.

### In vitro competitive profiling

The respective protein aliquot (0.4 mg/mL) was thawed on ice. 10 µL of protein were used per reaction and pipetted into a 1.5 mL micro reaction tube together with 9.6 µL of PBS as well as 0.4 µL of the competitive compound (10 mM). The proteins were incubated for 30 min at 25 °C and 400 rpm. Reaction of probe and ligand was done as described with 0.1 µL LS-probe (1 mM), ligand (1mM) and pyridine (5 M) per sample. The reacted probe was solved in 0.1 µL DMSO and pipetted to the proteins and incubated for 30 min at 37 °C and 400 rpm. Afterwards Click Chemistry was performed directly.

For dose down experiments the same procedure as for in vitro competitive profiling with LS-probes was performed. Final concentrations of 200 µM, 100 µM, 50 µM, 25 µM, 10 µM, 5 µM and 1 µM of the competitive compound were used with 5 µM of the reacted probe.

### Preparation of root extracts from red sage (Salvia miltiorrhiza)

For preparation of crude extracts, a protocol for purification of salvianolic acids was followed (Dong, J., Liu, Y., Liang, Z. & Wang, W. Investigation on ultrasound-assisted extraction of salvianolic acid B from Salvia miltiorrhiza root. Ultrason Sonochem 17, 61-5 (2010)). 10 g commercially available *Salvia miltiorrhiza* root powder was suspended in 200 mL 60 % ethanol (20 mL per 1 g powdered root) and subjected to sonication for 1 h. The mixture was filtered and the flow-through was concentrated *in vacuo* below 40 °C. Subsequent lyophilization yielded 4.916 g of crude extract as a brown solid. To further concentrate the extract, 4.5 g crude extract were resuspended in 50 mL H₂O and acidified with HCI to pH = 2. The mixture was extracted five times with ethyl acetate, the combined organic phases were dried over MgSO₄ and the solvent was evaporated, yielding 0.535 g of a dark red solid.

## Claims

1. A compound represented by the following general formula (1), 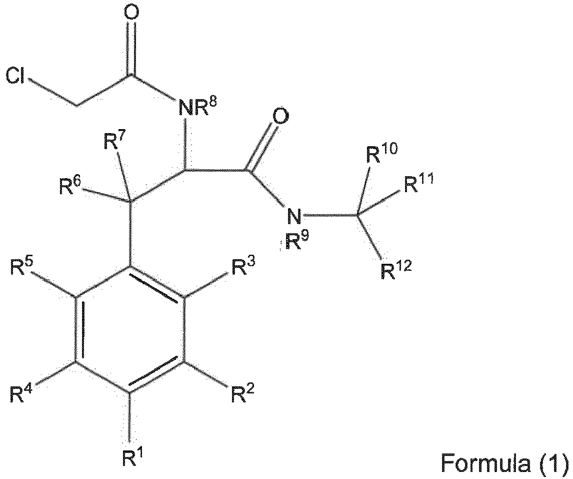
wherein one of R¹ to R⁵ is selected from the group consisting of an azide group, an alkyl group, a cycloalkyl group, an alkenyl group, a cycloalkenyl group, an alkynyl group, a biotin group, an aryl group, and a heteroaryl group, -NX¹X²,-OX³, -SX⁴, -C(O)X⁵, -C(O)NX⁶X⁷, -COOX⁸, and -SO₃X⁹, wherein X¹ to X⁹ are independently selected from the group consisting of a hydrogen atom, an alkyl group, a cycloalkyl group, an alkenyl group, a cycloalkenyl group, an alkynyl group, an aryl group, and a heteroaryl group, wherein the one of R¹ to R⁵ contains an azide group, an alkynyl group, a biotin group, or a fluorophoric group;
the remaining four of R¹ to R⁵ are independently selected from the group consisting of a hydrogen atom, an alkyl group, a cycloalkyl group, an alkenyl group, a cycloalkenyl group, an alkynyl group, an aryl group, a heteroaryl group, a halogen atom, -NE¹E², -NO₂, -CN, -OE³, -SE⁴, -C(O)E⁵, -C(O)NE⁶E⁷, -COOE⁸, and -SO₃E⁹, wherein E¹ to E⁹ are independently selected from the group consisting of a hydrogen atom, an alkyl group, a cycloalkyl group, an alkenyl group, a cycloalkenyl group, an alkynyl group, an aryl group, and a heteroaryl group, and wherein R¹ to R⁵ may bind to each other to form one or more rings;
R⁶, R⁷, and R¹⁰ to R¹² are independently selected from the group consisting of a hydrogen atom, an alkyl group, a cycloalkyl group, an alkenyl group, a cycloalkenyl group, an alkynyl group, an aryl group, a heteroaryl group, a halogen atom, -NA¹A², -NO₂, -CN, -OA³, -SA⁴, -C(O)A⁵, -C(O)NA⁶A⁷, -COOA⁸, and-SO₃A⁹, wherein A¹ to A⁹ are independently selected from the group consisting of a hydrogen atom, an alkyl group, a cycloalkyl group, an alkenyl group, a cycloalkenyl group, an alkynyl group, an aryl group, and a heteroaryl group, and wherein R⁶ and R⁷ and/or R¹⁰ to R¹² may bind to each other to form one or more rings; and
R⁸ and R⁹ are independently selected from the group consisting of a hydrogen atom, an alkyl group, a cycloalkyl group, an alkenyl group, a cycloalkenyl group, an alkynyl group, an aryl group, and a heteroaryl group;
or a pharmaceutically acceptable salt thereof; and
wherein the alkyl groups, the cycloalkyl groups, the alkenyl groups, the cycloalkenyl groups, the alkynyl groups, the aryl groups, and the heteroaryl groups may independently be (further) substituted or unsubstituted and the alkyl groups, the alkenyl groups, and the alkynyl groups may independently be branched or linear.

2. A pharmaceutical composition comprising the compound according to claim 1 or a pharmaceutically acceptable salt thereof, and optionally a pharmaceutically acceptable carrier, excipient or diluent.

3. The compound according to claim 1 or the pharmaceutical composition according to claim 2, wherein the compound or the pharmaceutical composition is for use in the treatment and prevention of an infection or a condition in a subject.

4. The compound for use according to claim 3 or the pharmaceutical composition for use according to claim 3, wherein the infection or condition is caused by a SARS coronavirus.

5. A production method for producing a compound represented by the following general formula (1), 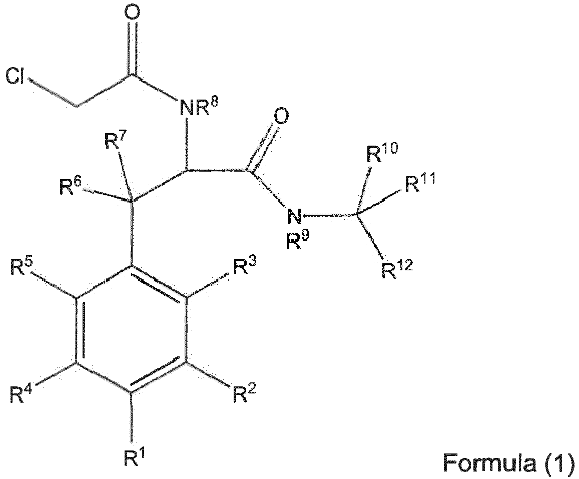 wherein the method comprises reacting a compound represented by the following general formula (2) 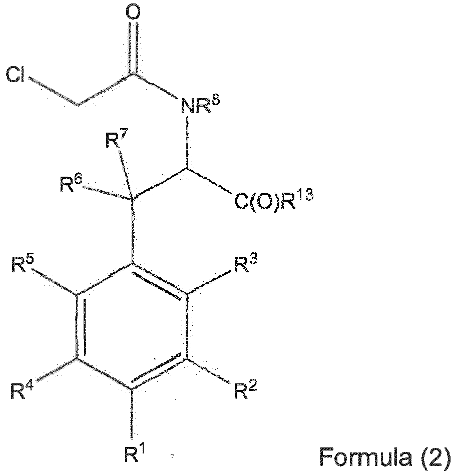 with a compound represented by the following general formula (3), 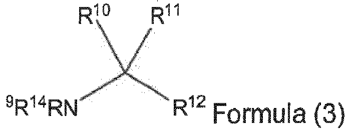
wherein one of R¹ to R⁵ is selected from the group consisting of an azide group, an alkyl group, a cycloalkyl group, an alkenyl group, a cycloalkenyl group, an alkynyl group, a biotin group, an aryl group, and a heteroaryl group, -NX¹X²,-OX³, -SX⁴, -C(O)X⁵, -C(O)NX⁶X⁷, -COOX⁸, and -SO₃X⁹, wherein X¹ to X⁹ are independently selected from the group consisting of a hydrogen atom, an alkyl group, a cycloalkyl group, an alkenyl group, a cycloalkenyl group, an alkynyl group, an aryl group, and a heteroaryl group, wherein the one of R¹ to R⁵ contains an azide group, an alkynyl group, a biotin group, or a fluorophoric group;
the remaining four of R¹ to R⁵ are independently selected from the group consisting of a hydrogen atom, an alkyl group, a cycloalkyl group, an alkenyl group, a cycloalkenyl group, an alkynyl group, an aryl group, a heteroaryl group, a halogen atom, -NE¹E², -NO₂, -CN, -OE³, -SE⁴, -C(O)E⁵, -C(O)NE⁶E⁷, -COOE⁸, and -SO₃E⁹, wherein E¹ to E⁹ are independently selected from the group consisting of a hydrogen atom, an alkyl group, a cycloalkyl group, an alkenyl group, a cycloalkenyl group, an alkynyl group, an aryl group, and a heteroaryl group, and wherein R¹ to R⁵ may bind to each other to form one or more rings; R⁶, R⁷, and R¹⁰ to R¹² are independently selected from the group consisting of a hydrogen atom, an alkyl group, a cycloalkyl group, an alkenyl group, a cycloalkenyl group, an alkynyl group, an aryl group, a heteroaryl group, a halogen atom, -NA¹A², -NO₂, -CN, -OA³, -SA⁴, -C(O)A⁵, -C(O)NA⁶A⁷, -COOA⁸, and-SO₃A⁹, wherein A¹ to A⁹ are independently selected from the group consisting of a hydrogen atom, an alkyl group, a cycloalkyl group, an alkenyl group, a cycloalkenyl group, an alkynyl group, an aryl group, and a heteroaryl group, and wherein R⁶ and R⁷ and/or R¹⁰ to R¹² may bind to each other to form one or more rings;
R⁸, R⁹, and R¹⁴ are independently selected from the group consisting of a hydrogen atom, an alkyl group, a cycloalkyl group, an alkenyl group, a cycloalkenyl group, an alkynyl group, an aryl group, and a heteroaryl group; and R¹³ is selected from the group consisting of a pentafluorophenyloxy group, a tetrafluorophenyloxy group, a trifluorophenyloxy group, a difluorophenyloxy group, a fluorophenyloxy group, a phenyloxy group, a pentafluorophenylthio group, a tetrafluorophenylthio group, a trifluorophenylthio group, a difluorophenylthio group, a fluorophenylthio group, a phenylthio group, a trinitrophenyloxy group, a dinitrophenyloxy group, a nitrophenyloxy group, a *N-*succinimidyloxy group, and a benzotriazolyloxy group; and
wherein the alkyl groups, the cycloalkyl groups, the alkenyl groups, the cycloalkenyl groups, the alkynyl groups, the aryl groups, and the heteroaryl groups may independently be (further) substituted or unsubstituted and the alkyl groups, the alkenyl groups, and the alkynyl groups may independently be branched or linear.

6. A screening method comprising a contacting step (a) of bringing at least one compound according to claim 1 or a salt thereof in contact with (wild type) protease(s) PL^{pro} and/or 3CL^{pro} of a SARS coronavirus.

7. The method according to claim 6, wherein the method comprises a further contacting step (a1) of bringing the at least one compound represented by the general formula (1) or a salt thereof in contact with modified protease(s) PL^{pro} and/or 3CL^{pro} of a SARS coronavirus.

8. The method according to claim 6 or 7, wherein the contacting step(s) is/are carried out *in vitro,* preferably in one or more cells.

9. The method according to any one of claims 6 to 8, wherein the method further comprises, after the contacting step (a), a step (b) of adding a compound selected from the group consisting of a fluorophore-azide, a fluorophore-alkyne, a biotin-azide, or a biotin-alkyne.

10. The method according to any one of claims 6 to 9, wherein the method further comprises an analyzing step (c) of analyzing as to whether the compound according to general formula (1) reacted with the protease(s).

11. The method according to any one of claims 6 to 10, wherein the method comprises a further contacting step (a0) of bringing the (wild type) protease PL^{pro} and/or 3CL^{pro} of a SARS coronavirus in contact with at least one compound to be screened before the contacting step (a) with the compound according to general formula (1).

12. The compound according to any one of claims 1, 3, and 4, the pharmaceutical composition according to any one of claims 2 to 4, the production method according to claim 5, and the screening method according to any one of claims 6 to 12, wherein each of R² to R¹¹ is a hydrogen atom.

13. The compound according to any one of claims 1, 3, 4, and 12, the pharmaceutical composition according to any one of claims 2 to 4 and 12, the production method according to claim 5 or 12, and the screening method according to any one of claims 6 to 12, wherein R¹² is selected from a n-propyl group, an iso-propyl group, an iso-butyl group, a 2,5-dichlorophenyl group, a 3,5-bis(trifluoromethyl)phenyl group, and a group of the following formulas (5) to (7)

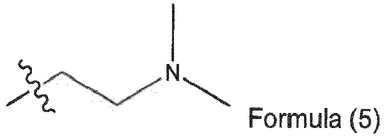

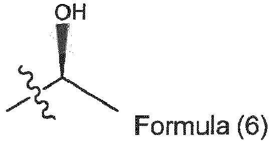

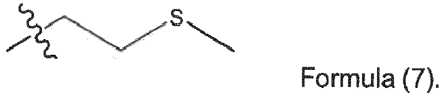

14. The compound according to any one of claims 1, 3, 4, 12, and 13, the pharmaceutical composition according to any one of claims 2 to 4, 12, and 13, the production method according to any one of claims 5, 12, and 13, and the screening method according to any one of claims 6 to 13, wherein the compound of the general formula (1) is selected from the compounds of the following formulas (10) to (18)


SEQUENCE LISTING

<110>  Universitaet Konstanz

<120>  Chemical Probes and Inhibitors for Polypeptides of SARS
       Coronaviruses

<130>  K6663EU

<160>  3

<170>  PatentIn version 3.5

<210>  1
<211>  5
<212>  PRT
<213>  Artificial Sequence

<220>
<223>  Arg-Leu-Arg-Gly-Gly-AMC


<220>
<221>  BINDING
<222>  (5)..(5)
<223>  7-amino-4-methylcoumarin substituted glycine

<400>  1

Arg Leu Arg Gly Gly
1               5


<210>  2
<211>  4
<212>  PRT
<213>  Artificial Sequence

<220>
<223>  Ala-Val-Leu-Gln-AMC


<220>
<221>  BINDING
<222>  (4)..(4)
<223>  7-amino-4-methylcoumarin substituted glutamine

<400>  2

Ala Val Leu Gln
1


<210>  3
<211>  4
<212>  PRT
<213>  Artificial Sequence

<220>
<223>  Ac-Thz-Tle-Leu-Gln-AMC


<220>

<221>  SITE
<222>  (1)..(1)
<223>  acetyl substituted L-thiazoline-4-carboxylic acid

<220>
<221>  SITE
<222>  (2)..(2)
<223>  t-Leucine

<220>
<221>  BINDING
<222>  (4)..(4)
<223>  7-amino-4-methylcoumarin substituted glutamine

<400>  3

Xaa Xaa Leu Gln
1